# EUROPEAN PATENT SPECIFICATION

(11) **EP 3 366 306 B1**
(45) Date of publication and mention of the grant of the patent: **29.09.2021**
(21) Application number: 17210566.0
(22) Date of filing: 25.12.2017
(51) Int. Cl.: A61K 35/28, G01N 33/68

(54) **USE OF SOME PEPTIDES IN DIAGNOSIS AND TREATMENT OF DIABETES, OBESITY AND METABOLIC DISEASES ASSOCIATED THERETO**
VERWENDUNG EINIGER PEPTIDE BEI DER DIAGNOSE UND BEHANDLUNG VON DIABETES, FETTLEIBIGKEIT UND DAMIT VERBUNDENEN STOFFWECHSELERKRANKUNGEN
UTILISATION DE CERTAINS PEPTIDES DANS LE DIAGNOSTIC ET LE TRAITEMENT DU DIABÈTE, DE L'OBÉSITÉ ET DES TROUBLES MÉTABOLIQUES ASSOCIÉS À CELUI-CI

(30) Priority: 23.12.2016 TR 201619391; 23.12.2016 TR 201619394; 23.12.2016 TR 201619399
(43) Date of publication of application: 29.08.2018
(73) Proprietor: Istanbul Üniversitesi Rektörlügü, 34452 Istanbul (TR)
(72) Inventor: Oktayoglu, Selda, 34134 Istanbul (TR); Bolkent, Sehnaz, 34134 Istanbul (TR); Uçar, Evren Önay, 34134 Istanbul (TR); Bas, Serap Sancar, 34134 Istanbul (TR); Karatug, Ayse, 34134 Istanbul (TR); Arda, Nazli, 34134 Istanbul (TR)
(74) Representative: Mutlu, Aydin

(56) References cited:
- WO-A1-2006/013114
- WO-A1-2007/058105
- DATABASE Geneseq [Online] 26 February 2004 (2004-02-26), "Novel human secreted and transmembrane protein SeqID 528.", XP002782004, retrieved from EBI accession no. GSP:ADF76853 Database accession no. ADF76853 & WO 03/072035 A2 (GENENTECH INC [US]) 4 September 2003 (2003-09-04)
- BROPHY ET AL: "RN181, a novel ubiquitin E3 ligase that interacts with the KVGFFKR motif of platelet integrin @a"I"I"b@b"3", BIOCHEMICAL AND BIOPHYSICAL RESEARCH COMMUNICATIONS, BIOCHEMICAL AND BIOPHYSICAL RESEARCH COMMUNICATIONS, AMSTERDAM, NL, vol. 369, no. 4, 10 March 2008 (2008-03-10), pages 1088-1093, XP022575468, ISSN: 0006-291X, DOI: 10.1016/J.BBRC.2008.02.142
- LIU MENG ET AL: "Mesenchymal stem cells: biology and clinical potential in type 1 diabetes therapy", JOURNAL OF CELLULAR AND MOLECULAR MEDICINE,, vol. 12, no. 4, 1 August 2008 (2008-08-01) , pages 1155-1168, XP002541175, DOI: 10.1111/J.1582-4934.2008.00288.X
- JIA-YU TAN ET AL: "Screening and verification of proteins that interact with HSPC238", ONCOLOGY REPORTS, vol. 34, no. 6, 18 September 2015 (2015-09-18), pages 3097-3103, XP055484170, ISSN: 1021-335X, DOI: 10.3892/or.2015.4289
- ROM OREN ET AL: "The role of E3 ubiquitin-ligases MuRF-1 and MAFbx in loss of skeletal muscle mass", FREE RADICAL BIOLOGY AND MEDICINE, ELSEVIER INC, US, vol. 98, 29 December 2015 (2015-12-29), pages 218-230, XP029671618, ISSN: 0891-5849, DOI: 10.1016/J.FREERADBIOMED.2015.12.031
- TAO BAI ET AL: "Diabetic cardiomyopathy: role of the E3 ubiquitin ligase", AMERICAN JOURNAL OF PHYSIOLOGY: ENDOCRINOLOGY AND METABOLISM., vol. 310, no. 7, 1 April 2016 (2016-04-01) , pages E473-E483, XP055484104, US ISSN: 0193-1849, DOI: 10.1152/ajpendo.00467.2015
- H. FUJITA ET AL: "The E3 ligase synoviolin controls body weight and mitochondrial biogenesis through negative regulation of PGC-1?", EMBO (EUROPEAN MOLECULAR BIOLOGY ORGANIZATION) JOURNAL, vol. 34, no. 8, 15 April 2015 (2015-04-15) , pages 1042-1055, XP055304897, DE ISSN: 0261-4189, DOI: 10.15252/embj.201489897
- SELDA GEZGINCI-OKTAYOGLU ET AL: "Involvement of dying beta cell originated messenger molecules in differentiation of pancreatic mesenchymal stem cells under glucotoxic and glucolipotoxic conditions", JOURNAL OF CELLULAR PHYSIOLOGY, vol. 233, no. 5, 28 November 2017 (2017-11-28), pages 4235-4244, XP055484677, US ISSN: 0021-9541, DOI: 10.1002/jcp.26242
- ARAVIND L ET AL: "Novel autoproteolytic and DNA-damage sensing components in the bacterial SOS response and oxidized methylcytosine-induced eukaryotic DNA demethylation systems", BIOLOGY DIRECT, BIOMED CENTRAL LTD, LO, vol. 8, no. 1, 15 August 2013 (2013-08-15) , page 20, XP021160719, ISSN: 1745-6150, DOI: 10.1186/1745-6150-8-20
- DATABASE Geneseq [Online] 1 January 2015 (2015-01-01), "Human inhibin-beta B pro-protein, SEQ 7.", XP002782046, retrieved from EBI accession no. GSP:BBP95339 Database accession no. BBP95339 & WO 2014/182676 A2 (SCHOLAR ROCK INC [US]) 13 November 2014 (2014-11-13)

### SEQUENCE LISTING

<110> ISTANBUL UNIVERSITESI
<120> Use of Some Peptides in Diagnosis and Treatment of Diabetes, Obesity and Metabolic Diseases Associated Thereto
<130> 06-17718-A
<160> 31
<170> PatentIn version 3.5
<210> 1
   <211> 153
   <212> PRT
   <213> Homo sapiens
<400> 1
   Met Ala Ser Tyr Phe Asp Glu His Asp Cys Glu Pro Ser Asp Pro Glu
   1 5 10 15
   Gln Glu Thr Arg Thr Asn Met Leu Leu Glu Leu Ala Arg Ser Leu Phe
   20 25 30
   Asn Arg Met Asp Phe Glu Asp Leu Gly Leu Val Val Asp Trp Asp His
   35 40 45
   His Leu Pro Pro Pro Ala Ala Lys Thr Val Val Glu Asn Leu Pro Arg
   50 55 60
   Thr Val Ile Arg Gly Ser Gln Ala Glu Leu Lys Cys Pro Val Cys Leu
   65 70 75 80
   Leu Glu Phe Glu Glu Glu Glu Thr Ala Ile Glu Met Pro Cys His His
   85 90 95
   Leu Phe His Ser Ser Cys Ile Leu Pro Trp Leu Ser Lys Thr Asn Ser
   100 105 110
   Cys Pro Leu Cys Arg Tyr Glu Leu Pro Thr Asp Asp Asp Thr Tyr Glu
   115 120 125
   Glu His Arg Arg Asp Lys Ala Arg Lys Gln Gln Gln Gln His Arg Leu
   130 135 140
   Glu Asn Leu His Gly Ala Met Tyr Thr
   145 150
<210> 2
   <211> 232
   <212> PRT
   <213> Homo sapiens
<400> 2
   Met Val Ser Ser Asp Val Tyr Gly Arg Asp Arg Met Thr Lys Ser Gly
   1 5 10 15
   Ser His Met Ser Val Leu Leu Val Gln Met Glu Trp Gly Leu Glu Leu
   20 25 30
   Ser Trp Gly Gly Ser Gln His Asp Arg Gln Ile Ile Asn Ala Arg Ala
   35 40 45
   Glu Ser Val Leu Ser Lys Pro Leu Phe Lys Glu Ser Met Arg Arg Gly
   50 55 60
   Arg Arg Cys Ala Ile Leu Thr Gln Gly Phe Tyr Glu Trp Arg Lys Ser
   65 70 75 80
   Thr Ser Gly Lys Gln Pro Tyr Phe Ile Tyr Met Pro Asp Asp Asp Glu
   85 90 95
   Asp Ser Lys Ser Ala Cys Ser Val Glu Cys Asp Ser Lys Leu Met Pro
   100 105 110
   Leu Ala Gly Leu Tyr Phe Pro Lys Lys Asp Lys Ser Pro Ala Tyr Thr
   115 120 125
   Ser Val Ile Ile Thr Val Asp Ser Ser Pro Gly Ile Glu Phe Ile His
   130 135 140
   Asp Arg Met Pro Ala Ile Leu Asp Thr Asp Glu Ala Val Leu Asn Trp
   145 150 155 160
   Leu Asp Cys Gly Gln Phe Gln Ser Asp Glu Val Val Lys Tyr Leu Arg
   165 170 175
   Pro Tyr Ala Ser Leu Arg Tyr His Ala Val Ser Thr Arg Val Asn His
   180 185 190
   Ser Ala Tyr Asp Asp Ala Glu Asn Ile Gln Pro Ile Asp Asp Ser Lys
   195 200 205
   Leu Ser Gly Glu Lys Arg Lys Ser Gln Pro Pro Leu Glu Ser Phe Phe
   210 215 220
   Ser Pro Lys Lys Glu Lys Leu Lys
   225 230
<210> 3
   <211> 407
   <212> PRT
   <213> Homo sapiens
<400> 3
   Met Asp Gly Leu Pro Gly Arg Ala Leu Gly Ala Ala Cys Leu Leu Leu
   1 5 10 15
   Leu Ala Ala Gly Trp Leu Gly Pro Glu Ala Trp Gly Ser Pro Thr Pro
   20 25 30
   Pro Pro Thr Pro Ala Ala Pro Pro Pro Pro Pro Pro Pro Gly Ser Pro
   35 40 45
   Gly Gly Ser Gln Asp Thr Cys Thr Ser Cys Gly Gly Phe Arg Arg Pro
   50 55 60
   Glu Glu Leu Gly Arg Val Asp Gly Asp Phe Leu Glu Ala Val Lys Arg
   65 70 75 80
   His Ile Leu Ser Arg Leu Gln Met Arg Gly Arg Pro Asn Ile Thr His
   85 90 95
   Ala Val Pro Lys Ala Ala Met Val Thr Ala Leu Arg Lys Leu His Ala
   100 105 110
   Gly Lys Val Arg Glu Asp Gly Arg Val Glu Ile Pro His Leu Asp Gly
   115 120 125
   His Ala Ser Pro Gly Ala Asp Gly Gln Glu Arg Val Ser Glu Ile Ile
   130 135 140
   Ser Phe Ala Glu Thr Asp Gly Leu Ala Ser Ser Arg Val Arg Leu Tyr
   145 150 155 160
   Phe Phe Ile Ser Asn Glu Gly Asn Gln Asn Leu Phe Val Val Gln Ala
   165 170 175
   Ser Leu Trp Leu Tyr Leu Lys Leu Leu Pro Tyr Val Leu Glu Lys Gly
   180 185 190
   Ser Arg Arg Lys Val Arg Val Lys Val Tyr Phe Gln Glu Gln Gly His
   195 200 205
   Gly Asp Arg Trp Asn Met Val Glu Lys Arg Val Asp Leu Lys Arg Ser
   210 215 220
   Gly Trp His Thr Phe Pro Leu Thr Glu Ala Ile Gln Ala Leu Phe Glu
   225 230 235 240
   Arg Gly Glu Arg Arg Leu Asn Leu Asp Val Gln Cys Asp Ser Cys Gln
   245 250 255
   Glu Leu Ala Val Val Pro Val Phe Val Asp Pro Gly Glu Glu Ser His
   260 265 270
   Arg Pro Phe Val Val Val Gln Ala Arg Leu Gly Asp Ser Arg His Arg
   275 280 285
   Ile Arg Lys Arg Gly Leu Glu Cys Asp Gly Arg Thr Asn Leu Cys Cys
   290 295 300
   Arg Gln Gln Phe Phe Ile Asp Phe Arg Leu Ile Gly Trp Asn Asp Trp
   305 310 315 320
   Ile Ile Ala Pro Thr Gly Tyr Tyr Gly Asn Tyr Cys Glu Gly Ser Cys
   325 330 335
   Pro Ala Tyr Leu Ala Gly Val Pro Gly Ser Ala Ser Ser Phe His Thr
   340 345 350
   Ala Val Val Asn Gln Tyr Arg Met Arg Gly Leu Asn Pro Gly Thr Val
   355 360 365
   Asn Ser Cys Cys Ile Pro Thr Lys Leu Ser Thr Met Ser Met Leu Tyr
   370 375 380
   Phe Asp Asp Glu Tyr Asn Ile Val Lys Arg Asp Val Pro Asn Met Ile
   385 390 395 400
   Val Glu Glu Cys Gly Cys Ala
   405

### SEQUENCE LISTING

<110> ISTANBUL UNIVERSITESI
<120> Use of Some Peptides in Diagnosis and Treatment of Diabetes, Obesity
   and Metabolic Diseases Associated Thereto
<130> 06-17718-A
<160> 31
<170> PatentIn version 3.5
<210> 1
   <211> 153
   <212> PRT
   <213> Homo sapiens
<400> 1
   Met Ala Ser Tyr Phe Asp Glu His Asp Cys Glu Pro Ser Asp Pro Glu
   1               5                   10                  15
   Gln Glu Thr Arg Thr Asn Met Leu Leu Glu Leu Ala Arg Ser Leu Phe
               20                  25                  30
   Asn Arg Met Asp Phe Glu Asp Leu Gly Leu Val Val Asp Trp Asp His
           35                  40                  45
   His Leu Pro Pro Pro Ala Ala Lys Thr Val Val Glu Asn Leu Pro Arg
       50                  55                  60
   Thr Val Ile Arg Gly Ser Gln Ala Glu Leu Lys Cys Pro Val Cys Leu
   65                  70                  75                  80
   Leu Glu Phe Glu Glu Glu Glu Thr Ala Ile Glu Met Pro Cys His His
                   85                  90                  95
   Leu Phe His Ser Ser Cys Ile Leu Pro Trp Leu Ser Lys Thr Asn Ser
               100                 105                 110
   Cys Pro Leu Cys Arg Tyr Glu Leu Pro Thr Asp Asp Asp Thr Tyr Glu
           115                 120                 125
   Glu His Arg Arg Asp Lys Ala Arg Lys Gln Gln Gln Gln His Arg Leu
       130                 135                 140
   Glu Asn Leu His Gly Ala Met Tyr Thr
   145                 150
<210> 2
   <211> 232
   <212> PRT
   <213> Homo sapiens
<400> 2
   Met Val Ser Ser Asp Val Tyr Gly Arg Asp Arg Met Thr Lys Ser Gly
   1               5                   10                  15
   Ser His Met Ser Val Leu Leu Val Gln Met Glu Trp Gly Leu Glu Leu
               20                  25                  30
   Ser Trp Gly Gly Ser Gln His Asp Arg Gln Ile Ile Asn Ala Arg Ala
           35                  40                  45
   Glu Ser Val Leu Ser Lys Pro Leu Phe Lys Glu Ser Met Arg Arg Gly
       50                  55                  60
   Arg Arg Cys Ala Ile Leu Thr Gln Gly Phe Tyr Glu Trp Arg Lys Ser
   65                  70                  75                  80
   Thr Ser Gly Lys Gln Pro Tyr Phe Ile Tyr Met Pro Asp Asp Asp Glu
                   85                  90                  95
   Asp Ser Lys Ser Ala Cys Ser Val Glu Cys Asp Ser Lys Leu Met Pro
               100                 105                 110
   Leu Ala Gly Leu Tyr Phe Pro Lys Lys Asp Lys Ser Pro Ala Tyr Thr
           115                 120                 125
   Ser Val Ile Ile Thr Val Asp Ser Ser Pro Gly Ile Glu Phe Ile His
       130                 135                 140
   Asp Arg Met Pro Ala Ile Leu Asp Thr Asp Glu Ala Val Leu Asn Trp
   145                 150                 155                 160
   Leu Asp Cys Gly Gln Phe Gln Ser Asp Glu Val Val Lys Tyr Leu Arg
                   165                 170                 175
   Pro Tyr Ala Ser Leu Arg Tyr His Ala Val Ser Thr Arg Val Asn His
               180                 185                 190
   Ser Ala Tyr Asp Asp Ala Glu Asn Ile Gln Pro Ile Asp Asp Ser Lys
           195                 200                 205
   Leu Ser Gly Glu Lys Arg Lys Ser Gln Pro Pro Leu Glu Ser Phe Phe
       210                 215                 220
   Ser Pro Lys Lys Glu Lys Leu Lys
   225                 230
<210> 3
   <211> 407
   <212> PRT
   <213> Homo sapiens
<400> 3
   Met Asp Gly Leu Pro Gly Arg Ala Leu Gly Ala Ala Cys Leu Leu Leu
   1               5                   10                  15
   Leu Ala Ala Gly Trp Leu Gly Pro Glu Ala Trp Gly Ser Pro Thr Pro
               20                  25                  30
   Pro Pro Thr Pro Ala Ala Pro Pro Pro Pro Pro Pro Pro Gly Ser Pro
           35                  40                  45
   Gly Gly Ser Gln Asp Thr Cys Thr Ser Cys Gly Gly Phe Arg Arg Pro
       50                  55                  60
   Glu Glu Leu Gly Arg Val Asp Gly Asp Phe Leu Glu Ala Val Lys Arg
   65                  70                  75                  80
   His Ile Leu Ser Arg Leu Gln Met Arg Gly Arg Pro Asn Ile Thr His
                   85                  90                  95
   Ala Val Pro Lys Ala Ala Met Val Thr Ala Leu Arg Lys Leu His Ala
               100                 105                 110
   Gly Lys Val Arg Glu Asp Gly Arg Val Glu Ile Pro His Leu Asp Gly
           115                 120                 125
   His Ala Ser Pro Gly Ala Asp Gly Gln Glu Arg Val Ser Glu Ile Ile
       130                 135                 140
   Ser Phe Ala Glu Thr Asp Gly Leu Ala Ser Ser Arg Val Arg Leu Tyr
   145                 150                 155                 160
   Phe Phe Ile Ser Asn Glu Gly Asn Gln Asn Leu Phe Val Val Gln Ala
                   165                 170                 175
   Ser Leu Trp Leu Tyr Leu Lys Leu Leu Pro Tyr Val Leu Glu Lys Gly
               180                 185                 190
   Ser Arg Arg Lys Val Arg Val Lys Val Tyr Phe Gln Glu Gln Gly His
           195                 200                 205
   Gly Asp Arg Trp Asn Met Val Glu Lys Arg Val Asp Leu Lys Arg Ser
       210                 215                 220
   Gly Trp His Thr Phe Pro Leu Thr Glu Ala Ile Gln Ala Leu Phe Glu
   225 230 235 240
   Arg Gly Glu Arg Arg Leu Asn Leu Asp Val Gln Cys Asp Ser Cys Gln
   245 250 255
   Glu Leu Ala Val Val Pro Val Phe Val Asp Pro Gly Glu Glu Ser His
   260 265 270
   Arg Pro Phe Val Val Val Gln Ala Arg Leu Gly Asp Ser Arg His Arg
   275 280 285
   Ile Arg Lys Arg Gly Leu Glu Cys Asp Gly Arg Thr Asn Leu Cys Cys
   290 295 300
   Arg Gln Gln Phe Phe Ile Asp Phe Arg Leu Ile Gly Trp Asn Asp Trp
   305 310 315 320
   Ile Ile Ala Pro Thr Gly Tyr Tyr Gly Asn Tyr Cys Glu Gly Ser Cys
   325 330 335
   Pro Ala Tyr Leu Ala Gly Val Pro Gly Ser Ala Ser Ser Phe His Thr
   340 345 350
   Ala Val Val Asn Gln Tyr Arg Met Arg Gly Leu Asn Pro Gly Thr Val
   355 360 365
   Asn Ser Cys Cys Ile Pro Thr Lys Leu Ser Thr Met Ser Met Leu Tyr
   370 375 380
   Phe Asp Asp Glu Tyr Asn Ile Val Lys Arg Asp Val Pro Asn Met Ile
   385 390 395 400
   Val Glu Glu Cys Gly Cys Ala
   405
<210> 4
   <211> 384
   <212> PRT
   <213> Homo sapiens
<400> 4
<210> 5
   <211> 324
   <212> PRT
   <213> Homo sapiens
<400> 5
   Met Asp Ala Ala Leu Leu Leu Asn Val Glu Gly Val Lys Lys Thr Ile
   1               5                   10                  15
   Leu His Gly Gly Thr Gly Glu Leu Pro Asn Phe Ile Thr Gly Ser Arg
               20                  25                  30
   His Thr Gly Val Tyr Pro Ile Leu Ser Arg Ser Leu Arg Gln Met Ala
           35                  40                  45
   Gln Gly Lys Asp Pro Thr Glu Trp His Val His Thr Cys Gly Leu Ala
       50                  55                  60
   Asn Met Phe Ala Tyr His Thr Leu Gly Tyr Glu Asp Leu Asp Glu Leu
   65                  70                  75                  80
   Gln Lys Glu Pro Gln Pro Leu Val Phe Val Ile Glu Leu Leu Gln Val
                   85                  90                  95
   Asp Ala Pro Ser Asp Tyr Gln Arg Glu Thr Trp Asn Leu Ser Asn His
               100                 105                 110
   Glu Lys Met Lys Ala Val Pro Val Leu His Gly Glu Gly Asn Arg Leu
   115 120 125
   Phe Lys Leu Gly Arg Tyr Glu Glu Ala Ser Ser Lys Tyr Gln Glu Ala
   130 135 140
   Ile Ile Cys Leu Arg Asn Leu Gln Thr Lys Glu Lys Pro Trp Glu Val
   145 150 155 160
   Gln Trp Leu Lys Leu Glu Lys Met Ile Asn Thr Leu Ile Leu Asn Tyr
   165 170 175
   Cys Gln Cys Leu Leu Lys Lys Glu Glu Tyr Tyr Glu Val Leu Glu His
   180 185 190
   Thr Ser Asp Ile Leu Arg His His Pro Gly Ile Val Lys Ala Tyr Tyr
   195 200 205
   Val Arg Ala Arg Ala His Ala Glu Val Trp Asn Glu Ala Glu Ala Lys
   210 215 220
   Ala Asp Leu Gln Lys Val Leu Glu Leu Glu Pro Ser Met Gln Lys Ala
   225 230 235 240
   Val Arg Arg Glu Leu Arg Leu Leu Glu Asn Arg Met Ala Glu Lys Gln
   245 250 255
   Glu Glu Glu Arg Leu Arg Cys Arg Asn Met Leu Ser Gln Gly Ala Thr
   260 265 270
   Gln Pro Pro Ala Glu Pro Pro Thr Glu Pro Pro Ala Gln Ser Ser Thr
   275 280 285
   Glu Pro Pro Ala Glu Pro Pro Thr Ala Pro Ser Ala Glu Leu Ser Ala
   290 295 300
   Gly Pro Pro Ala Glu Pro Ala Thr Glu Pro Pro Pro Ser Pro Gly His
   305 310 315 320
   Ser Leu Gln His
<210> 6
   <211> 321
   <212> PRT
   <213> Homo sapiens
<400> 6
   Met Asp Ala Ala Leu Leu Leu Asn Val Glu Gly Val Lys Lys Thr Ile
   1 5 10 15
   Leu His Gly Gly Thr Gly Glu Leu Pro Asn Phe Ile Thr Gly Ser Arg
   20 25 30
   Val Ile Phe His Phe Arg Thr Met Lys Cys Asp Glu Glu Arg Thr Val
   35 40 45
   Ile Asp Asp Ser Arg Gln Val Gly Gln Pro Met His Ile Ile Ile Gly
   50 55 60
   Asn Met Phe Lys Leu Glu Val Trp Glu Ile Leu Leu Thr Ser Met Arg
   65 70 75 80
   Val His Glu Val Ala Glu Phe Trp Cys Asp Thr Ile Val Asp Ala Pro
   85 90 95
   Ser Asp Tyr Gln Arg Glu Thr Trp Asn Leu Ser Asn His Glu Lys Met
   100 105 110
   Lys Ala Val Pro Val Leu His Gly Glu Gly Asn Arg Leu Phe Lys Leu
   115 120 125
   Gly Arg Tyr Glu Glu Ala Ser Ser Lys Tyr Gln Glu Ala Ile Ile Cys
   130 135 140
   Leu Arg Asn Leu Gln Thr Lys Glu Lys Pro Trp Glu Val Gln Trp Leu
   145 150 155 160
   Lys Leu Glu Lys Met Ile Asn Thr Leu Ile Leu Asn Tyr Cys Gln Cys
   165 170 175
   Leu Leu Lys Lys Glu Glu Tyr Tyr Glu Val Leu Glu His Thr Ser Asp
   180 185 190
   Ile Leu Arg His His Pro Gly Ile Val Lys Ala Tyr Tyr Val Arg Ala
   195 200 205
   Arg Ala His Ala Glu Val Trp Asn Glu Ala Glu Ala Lys Ala Asp Leu
   210 215 220
   Gln Lys Val Leu Glu Leu Glu Pro Ser Met Gln Lys Ala Val Arg Arg
   225 230 235 240
   Glu Leu Arg Leu Leu Glu Asn Arg Met Ala Glu Lys Gln Glu Glu Glu
   245 250 255
   Arg Leu Arg Cys Arg Asn Met Leu Ser Gln Gly Ala Thr Gln Pro Pro
   260 265 270
   Ala Glu Pro Pro Thr Glu Pro Pro Ala Gln Ser Ser Thr Glu Pro Pro
   275 280 285
   Ala Glu Pro Pro Thr Ala Pro Ser Ala Glu Leu Ser Ala Gly Pro Pro
   290 295 300
   Ala Glu Pro Ala Thr Glu Pro Pro Pro Ser Pro Gly His Ser Leu Gln
   305 310 315 320
   His
<210> 7
   <211> 362
   <212> PRT
   <213> Homo sapiens
<400> 7
   Met Asp Ala Ala Leu Leu Leu Asn Val Glu Gly Val Lys Lys Thr Ile
   1 5 10 15
   Leu His Gly Gly Thr Gly Glu Leu Pro Asn Phe Ile Thr Gly Ser Arg
   20 25 30
   Val Gly Gln Pro Met His Ile Ile Ile Gly Asn Met Phe Lys Leu Glu
   35 40 45
   Val Trp Glu Ile Leu Leu Thr Ser Met Arg Val His Glu Val Ala Glu
   50 55 60
   Phe Trp Cys Asp Thr Ile His Thr Gly Val Tyr Pro Ile Leu Ser Arg
   65 70 75 80
   Ser Leu Arg Gln Met Ala Gln Gly Lys Asp Pro Thr Glu Trp His Val
   85 90 95
   His Thr Cys Gly Leu Ala Asn Met Phe Ala Tyr His Thr Leu Gly Tyr
   100 105 110
   Glu Asp Leu Asp Glu Leu Gln Lys Glu Pro Gln Pro Leu Val Phe Val
   115 120 125
   Ile Glu Leu Leu Gln Val Asp Ala Pro Ser Asp Tyr Gln Arg Glu Thr
   130 135 140
   Trp Asn Leu Ser Asn His Glu Lys Met Lys Ala Val Pro Val Leu His
   145                 150                 155                 160
   Gly Glu Gly Asn Arg Leu Phe Lys Leu Gly Arg Tyr Glu Glu Ala Ser
                   165                 170                 175
   Ser Lys Tyr Gln Glu Ala Ile Ile Cys Leu Arg Asn Leu Gln Thr Lys
               180                 185                 190
   Glu Lys Pro Trp Glu Val Gln Trp Leu Lys Leu Glu Lys Met Ile Asn
           195                 200                 205
   Thr Leu Ile Leu Asn Tyr Cys Gln Cys Leu Leu Lys Lys Glu Glu Tyr
       210                 215                 220
   Tyr Glu Val Leu Glu His Thr Ser Asp Ile Leu Arg His His Pro Gly
   225                 230                 235                 240
   Ile Val Lys Ala Tyr Tyr Val Arg Ala Arg Ala His Ala Glu Val Trp
                   245                 250                 255
   Asn Glu Ala Glu Ala Lys Ala Asp Leu Gln Lys Val Leu Glu Leu Glu
               260                 265                 270
   Pro Ser Met Gln Lys Ala Val Arg Arg Glu Leu Arg Leu Leu Glu Asn
           275                 280                 285
   Arg Met Ala Glu Lys Gln Glu Glu Glu Arg Leu Arg Cys Arg Asn Met
       290                 295                 300
   Leu Ser Gln Gly Ala Thr Gln Pro Pro Ala Glu Pro Pro Thr Glu Pro
   305                 310                 315                 320
   Pro Ala Gln Ser Ser Thr Glu Pro Pro Ala Glu Pro Pro Thr Ala Pro
                   325                 330                 335
   Ser Ala Glu Leu Ser Ala Gly Pro Pro Ala Glu Pro Ala Thr Glu Pro
               340                 345                 350
   Pro Pro Ser Pro Gly His Ser Leu Gln His
           355                 360
<210> 8
   <211> 360
   <212> PRT
   <213> Homo sapiens
<400> 8
   Met Asp Ala Ala Leu Leu Leu Asn Val Glu Gly Val Lys Lys Thr Ile
   1               5                   10                  15
   Leu His Gly Gly Thr Gly Glu Leu Pro Asn Phe Ile Thr Gly Ser Arg
               20                  25                  30
   Val Ile Phe His Phe Arg Thr Met Lys Cys Asp Glu Glu Arg Thr Val
           35                  40                  45
   Ile Asp Asp Ser Arg Gln Val Gly Gln Pro Met His Ile Ile Ile Gly
       50                  55                  60
   Asn Met Phe Lys Leu Glu Val Trp Glu Ile Leu Leu Thr Ser Met Arg
   65                  70                  75                  80
   Val His Glu Val Ala Glu Phe Trp Cys Asp Thr Ile His Thr Gly Val
                   85                  90                  95
   Tyr Pro Ile Leu Ser Arg Ser Leu Arg Gln Met Ala Gln Gly Lys Asp
               100                 105                 110
   Pro Thr Glu Trp His Val His Thr Cys Gly Leu Ala Asn Met Phe Ala
           115                 120                 125
   Tyr His Thr Leu Gly Tyr Glu Asp Leu Asp Glu Leu Gln Lys Glu Pro
       130                 135                 140
   Gln Pro Leu Val Phe Val Ile Glu Leu Leu Gln Val Asp Ala Pro Ser
   145                 150                 155                 160
   Asp Tyr Gln Arg Glu Thr Trp Asn Leu Ser Asn His Glu Lys Met Lys
                   165                 170                 175
   Ala Val Pro Val Leu His Gly Glu Gly Asn Arg Leu Phe Lys Leu Gly
               180                 185                 190
   Arg Tyr Glu Glu Ala Ser Ser Lys Tyr Gln Glu Ala Ile Ile Cys Leu
           195                 200                 205
   Arg Asn Leu Gln Thr Lys Cys Leu Leu Lys Lys Glu Glu Tyr Tyr Glu
       210                 215                 220
   Val Leu Glu His Thr Ser Asp Ile Leu Arg His His Pro Gly Ile Val
   225                 230                 235                 240
   Lys Ala Tyr Tyr Val Arg Ala Arg Ala His Ala Glu Val Trp Asn Glu
                   245                 250                 255
   Ala Glu Ala Lys Ala Asp Leu Gln Lys Val Leu Glu Leu Glu Pro Ser
   260 265 270
   Met Gln Lys Ala Val Arg Arg Glu Leu Arg Leu Leu Glu Asn Arg Met
   275 280 285
   Ala Glu Lys Gln Glu Glu Glu Arg Leu Arg Cys Arg Asn Met Leu Ser
   290 295 300
   Gln Gly Ala Thr Gln Pro Pro Ala Glu Pro Pro Thr Glu Pro Pro Ala
   305 310 315 320
   Gln Ser Ser Thr Glu Pro Pro Ala Glu Pro Pro Thr Ala Pro Ser Ala
   325 330 335
   Glu Leu Ser Ala Gly Pro Pro Ala Glu Pro Ala Thr Glu Pro Pro Pro
   340 345 350
   Ser Pro Gly His Ser Leu Gln His
   355 360
<210> 9
   <211> 206
   <212> PRT
   <213> Homo sapiens
<400> 9
   Met Ala Ala Asn Lys Ser Lys Gly Gln Ser Ser Leu Ala Leu His Lys
   1 5 10 15
   Val Ile Met Val Gly Ser Gly Gly Val Gly Lys Ser Ala Leu Thr Leu
   20 25 30
   Gln Phe Met Tyr Asp Glu Phe Val Glu Asp Tyr Glu Pro Thr Lys Ala
   35 40 45
   Asp Ser Tyr Arg Lys Lys Val Val Leu Asp Gly Glu Glu Val Gln Ile
   50 55 60
   Asp Ile Leu Asp Thr Ala Gly Gln Glu Asp Tyr Ala Ala Ile Arg Asp
   65 70 75 80
   Asn Tyr Phe Arg Ser Gly Glu Gly Phe Leu Leu Val Phe Ser Ile Thr
   85 90 95
   Glu His Glu Ser Phe Thr Ala Thr Ala Glu Phe Arg Glu Gln Ile Leu
   100 105 110
   Arg Val Lys Ala Glu Glu Asp Lys Ile Pro Leu Leu Val Val Gly Asn
           115                 120                 125
   Lys Ser Asp Leu Glu Glu Arg Arg Gln Val Pro Val Glu Glu Ala Arg
       130                 135                 140
   Ser Lys Ala Glu Glu Trp Gly Val Gln Tyr Val Glu Thr Ser Ala Lys
   145                 150                 155                 160
   Thr Arg Ala Asn Val Asp Lys Val Phe Phe Asp Leu Met Arg Glu Ile
                   165                 170                 175
   Arg Thr Lys Lys Met Ser Glu Asn Lys Asp Lys Asn Gly Lys Lys Ser
               180                 185                 190
   Ser Lys Asn Lys Lys Ser Phe Lys Glu Arg Cys Cys Leu Leu
           195                 200                 205
<210> 10
   <211> 228
   <212> PRT
   <213> Homo sapiens
<400> 10
   Met Lys Gln Arg Gln Ser Ala Leu Gln Trp Val Ile Cys Val Ser Gln
   1               5                   10                  15
   Pro Gln Lys Thr Ser Glu Met Ala Ala Asn Lys Ser Lys Gly Gln Ser
               20                  25                  30
   Ser Leu Ala Leu His Lys Val Ile Met Val Gly Ser Gly Gly Val Gly
           35                  40                  45
   Lys Ser Ala Leu Thr Leu Gln Phe Met Tyr Asp Glu Phe Val Glu Asp
       50                  55                  60
   Tyr Glu Pro Thr Lys Ala Asp Ser Tyr Arg Lys Lys Val Val Leu Asp
   65                  70                  75                  80
   Gly Glu Glu Val Gln Ile Asp Ile Leu Asp Thr Ala Gly Gln Glu Asp
                   85                  90                  95
   Tyr Ala Ala Ile Arg Asp Asn Tyr Phe Arg Ser Gly Glu Gly Phe Leu
               100                 105                 110
   Leu Val Phe Ser Ile Thr Glu His Glu Ser Phe Thr Ala Thr Ala Glu
           115                 120                 125
   Phe Arg Glu Gln Ile Leu Arg Val Lys Ala Glu Glu Asp Lys Ile Pro
       130                 135                 140
   Leu Leu Val Val Gly Asn Lys Ser Asp Leu Glu Glu Arg Arg Gln Val
   145                 150                 155                 160
   Pro Val Glu Glu Ala Arg Ser Lys Ala Glu Glu Trp Gly Val Gln Tyr
                   165                 170                 175
   Val Glu Thr Ser Ala Lys Thr Arg Ala Asn Val Asp Lys Val Phe Phe
               180                 185                 190
   Asp Leu Met Arg Glu Ile Arg Thr Lys Lys Met Ser Glu Asn Lys Asp
           195                 200                 205
   Lys Asn Gly Lys Lys Ser Ser Lys Asn Lys Lys Ser Phe Lys Glu Arg
       210                 215                 220
   Cys Cys Leu Leu
   225
<210> 11
   <211> 227
   <212> PRT
   <213> Homo sapiens
<400> 11
   Met Ala Ala Asn Lys Ser Lys Gly Gln Ser Ser Leu Ala Leu His Lys
   1               5                   10                  15
   Val Ile Met Val Gly Ser Gly Gly Val Gly Lys Ser Ala Leu Thr Leu
               20                  25                  30
   Gln Phe Met Asn Val Ser Lys Ser Leu Ala Tyr Asp Lys Lys Lys Tyr
           35                  40                  45
   Thr Ala Asn Lys Lys Val Glu Gly Ile Leu Glu Phe Val Glu Asp Tyr
       50                  55                  60
   Glu Pro Thr Lys Ala Asp Ser Tyr Arg Lys Lys Val Val Leu Asp Gly
   65                  70                  75                  80
   Glu Glu Val Gln Ile Asp Ile Leu Asp Thr Ala Gly Gln Glu Asp Tyr
                   85                  90                  95
   Ala Ala Ile Arg Asp Asn Tyr Phe Arg Ser Gly Glu Gly Phe Leu Leu
               100                 105                 110
   Val Phe Ser Ile Thr Glu His Glu Ser Phe Thr Ala Thr Ala Glu Phe
   115 120 125
   Arg Glu Gln Ile Leu Arg Val Lys Ala Glu Glu Asp Lys Ile Pro Leu
   130 135 140
   Leu Val Val Gly Asn Lys Ser Asp Leu Glu Glu Arg Arg Gln Val Pro
   145 150 155 160
   Val Glu Glu Ala Arg Ser Lys Ala Glu Glu Trp Gly Val Gln Tyr Val
   165 170 175
   Glu Thr Ser Ala Lys Thr Arg Ala Asn Val Asp Lys Val Phe Phe Asp
   180 185 190
   Leu Met Arg Glu Ile Arg Thr Lys Lys Met Ser Glu Asn Lys Asp Lys
   195 200 205
   Asn Gly Lys Lys Ser Ser Lys Asn Lys Lys Ser Phe Lys Glu Arg Cys
   210 215 220
   Cys Leu Leu
   225
<210> 12
   <211> 424
   <212> PRT
   <213> Homo sapiens
<400> 12
<210> 13
   <211> 348
   <212> PRT
   <213> Homo sapiens
<400> 13
   Met Ser Asp His Gly Asp Val Ser Leu Pro Pro Glu Asp Arg Val Arg
   1 5 10 15
   Ala Leu Ser Gln Leu Gly Ser Ala Val Glu Val Asn Glu Asp Ile Pro
   20 25 30
   Pro Arg Arg Tyr Phe Arg Ser Gly Val Glu Ile Ile Arg Met Ala Ser
   35 40 45
   Ile Tyr Ser Glu Glu Gly Asn Ile Glu His Ala Phe Ile Leu Tyr Asn
   50 55 60
   Lys Tyr Ile Thr Leu Phe Ile Glu Lys Leu Pro Lys His Arg Asp Tyr
   65 70 75 80
   Lys Ser Ala Val Ile Pro Glu Lys Lys Asp Thr Val Lys Lys Leu Lys
   85 90 95
   Glu Ile Ala Phe Pro Lys Ala Glu Glu Leu Lys Ala Glu Leu Leu Lys
   100 105 110
   Arg Tyr Thr Lys Glu Tyr Thr Glu Tyr Asn Glu Glu Lys Lys Lys Glu
   115 120 125
   Ala Glu Glu Leu Ala Arg Asn Met Ala Ile Gln Gln Glu Leu Glu Lys
   130 135 140
   Glu Lys Gln Arg Val Ala Gln Gln Lys Gln Gln Gln Leu Glu Gln Glu
   145 150 155 160
   Gln Phe His Ala Phe Glu Glu Met Ile Arg Asn Gln Glu Leu Glu Lys
   165 170 175
   Glu Arg Leu Lys Ile Val Gln Glu Phe Gly Lys Val Asp Pro Gly Leu
   180 185 190
   Gly Gly Pro Leu Val Pro Asp Leu Glu Lys Pro Ser Leu Asp Val Phe
   195 200 205
   Pro Thr Leu Thr Val Ser Ser Ile Gln Pro Ser Asp Cys His Thr Thr
   210 215 220
   Val Arg Pro Ala Lys Pro Pro Val Val Asp Arg Ser Leu Lys Pro Gly
   225 230 235 240
   Ala Leu Ser Asn Ser Glu Ser Ile Pro Thr Ile Asp Gly Leu Arg His
   245 250 255
   Val Val Val Pro Gly Arg Leu Cys Pro Gln Phe Leu Gln Leu Ala Ser
   260 265 270
   Ala Asn Thr Ala Arg Gly Val Glu Thr Cys Gly Ile Leu Cys Gly Lys
   275 280 285
   Leu Met Arg Asn Glu Phe Thr Ile Thr His Val Leu Ile Pro Lys Gln
   290 295 300
   Ser Ala Gly Ser Asp Tyr Cys Asn Thr Glu Asn Glu Glu Glu Leu Phe
   305 310 315 320
   Leu Ile Gln Asp Gln Gln Gly Leu Ile Thr Leu Gly Trp Ile His Val
   325 330 335
   Glu Thr Leu Trp Ser Leu Lys Ser Leu His Ala Pro
   340 345
<210> 14
   <211> 423
   <212> PRT
   <213> Homo sapiens
<400> 14
   Met Ser Ser Gln Val Val Gly Ile Glu Pro Leu Tyr Ile Lys Ala Glu
   1 5 10 15
   Pro Ala Ser Pro Asp Ser Pro Lys Gly Ser Ser Glu Thr Glu Thr Glu
               20                  25                  30
   Pro Pro Val Ala Leu Ala Pro Gly Pro Ala Pro Thr Arg Cys Leu Pro
           35                  40                  45
   Gly His Lys Glu Glu Glu Asp Gly Glu Gly Ala Gly Pro Gly Glu Gln
       50                  55                  60
   Gly Gly Gly Lys Leu Val Leu Ser Ser Leu Pro Lys Arg Leu Cys Leu
   65                  70                  75                  80
   Val Cys Gly Asp Val Ala Ser Gly Tyr His Tyr Gly Val Ala Ser Cys
                   85                  90                  95
   Glu Ala Cys Lys Ala Phe Phe Lys Arg Thr Ile Gln Gly Ser Ile Glu
               100                 105                 110
   Tyr Ser Cys Pro Ala Ser Asn Glu Cys Glu Ile Thr Lys Arg Arg Arg
           115                 120                 125
   Lys Ala Cys Gln Ala Cys Arg Phe Thr Lys Cys Leu Arg Val Gly Met
       130                 135                 140
   Leu Lys Glu Gly Val Arg Leu Asp Arg Val Arg Gly Gly Arg Gln Lys
   145                 150                 155                 160
   Tyr Lys Arg Arg Pro Glu Val Asp Pro Leu Pro Phe Pro Gly Pro Phe
                   165                 170                 175
   Pro Ala Gly Pro Leu Ala Val Ala Gly Gly Pro Arg Lys Thr Ala Ala
               180                 185                 190
   Pro Val Asn Ala Leu Val Ser His Leu Leu Val Val Glu Pro Glu Lys
           195                 200                 205
   Leu Tyr Ala Met Pro Asp Pro Ala Gly Pro Asp Gly His Leu Pro Ala
       210                 215                 220
   Val Ala Thr Leu Cys Asp Leu Phe Asp Arg Glu Ile Val Val Thr Ile
   225                 230                 235                 240
   Ser Trp Ala Lys Ser Ile Pro Gly Phe Ser Ser Leu Ser Leu Ser Asp
                   245                 250                 255
   Gln Met Ser Val Leu Gln Ser Val Trp Met Glu Val Leu Val Leu Gly
   260 265 270
   Val Ala Gln Arg Ser Leu Pro Leu Gln Asp Glu Leu Ala Phe Ala Glu
   275 280 285
   Asp Leu Val Leu Asp Glu Glu Gly Ala Arg Ala Ala Gly Leu Gly Glu
   290 295 300
   Leu Gly Ala Ala Leu Leu Gln Leu Val Arg Arg Leu Gln Ala Leu Arg
   305 310 315 320
   Leu Glu Arg Glu Glu Tyr Val Leu Leu Lys Ala Leu Ala Leu Ala Asn
   325 330 335
   Ser Asp Ser Val His Ile Glu Asp Ala Glu Ala Val Glu Gln Leu Arg
   340 345 350
   Glu Ala Leu His Glu Ala Leu Leu Glu Tyr Glu Ala Gly Arg Ala Gly
   355 360 365
   Pro Gly Gly Gly Ala Glu Arg Arg Arg Ala Gly Arg Leu Leu Leu Thr
   370 375 380
   Leu Pro Leu Leu Arg Gln Thr Ala Gly Lys Val Leu Ala His Phe Tyr
   385 390 395 400
   Gly Val Lys Leu Glu Gly Lys Val Pro Met His Lys Leu Phe Leu Glu
   405 410 415
   Met Leu Glu Ala Met Met Asp
   420
<210> 15
   <211> 421
   <212> PRT
   <213> Homo sapiens
<400> 15
   Met Ser Ser Gln Val Val Gly Ile Glu Pro Leu Tyr Ile Lys Ala Glu
   1               5                   10                  15
   Pro Ala Ser Pro Asp Ser Pro Lys Gly Ser Ser Glu Thr Glu Thr Glu
               20                  25                  30
   Pro Pro Val Ala Leu Ala Pro Gly Pro Ala Pro Thr Arg Cys Leu Pro
           35                  40                  45
   Gly His Lys Glu Glu Glu Asp Gly Glu Gly Ala Gly Pro Gly Glu Gln
   50 55 60
   Gly Gly Gly Lys Leu Val Leu Ser Ser Leu Pro Lys Arg Leu Cys Leu
   65 70 75 80
   Val Cys Gly Asp Val Ala Ser Gly Tyr His Tyr Gly Val Ala Ser Cys
   85 90 95
   Glu Ala Cys Lys Ala Phe Phe Lys Arg Thr Ile Gln Gly Ser Ile Glu
   100 105 110
   Tyr Ser Cys Pro Ala Ser Asn Glu Cys Glu Ile Thr Lys Arg Arg Arg
   115 120 125
   Lys Ala Cys Gln Ala Cys Arg Phe Thr Lys Cys Leu Arg Val Gly Met
   130 135 140
   Leu Lys Glu Gly Val Arg Leu Asp Arg Val Arg Gly Gly Arg Gln Lys
   145 150 155 160
   Tyr Lys Arg Arg Pro Glu Val Asp Pro Leu Pro Phe Pro Gly Pro Phe
   165 170 175
   Pro Ala Gly Pro Leu Ala Val Ala Gly Gly Pro Arg Lys Thr Ala Pro
   180 185 190
   Val Asn Ala Leu Val Ser Leu Leu Val Val Glu Pro Glu Lys Leu Tyr
   195 200 205
   Ala Met Pro Asp Pro Ala Gly Pro Asp Gly His Leu Pro Ala Val Ala
   210 215 220
   Thr Leu Cys Asp Leu Phe Asp Arg Glu Ile Val Val Thr Ile Ser Trp
   225 230 235 240
   Ala Lys Ser Ile Pro Gly Phe Ser Ser Leu Ser Leu Ser Asp Gln Met
   245 250 255
   Ser Val Leu Gln Ser Val Trp Met Glu Val Leu Val Leu Gly Val Ala
   260 265 270
   Gln Arg Ser Leu Pro Leu Gln Asp Glu Leu Ala Phe Ala Glu Asp Leu
   275 280 285
   Val Leu Asp Glu Glu Gly Ala Arg Ala Ala Gly Leu Gly Glu Leu Gly
   290 295 300
   Ala Ala Leu Leu Gln Leu Val Arg Arg Leu Gln Ala Leu Arg Leu Glu
   305                 310                 315                 320
   Arg Glu Glu Tyr Val Leu Leu Lys Ala Leu Ala Leu Ala Asn Ser Asp
                   325                 330                 335
   Ser Val His Ile Glu Asp Ala Glu Ala Val Glu Gln Leu Arg Glu Ala
               340                 345                 350
   Leu His Glu Ala Leu Leu Glu Tyr Glu Ala Gly Arg Ala Gly Pro Gly
           355                 360                 365
   Gly Gly Ala Glu Arg Arg Arg Ala Gly Arg Leu Leu Leu Thr Leu Pro
       370                 375                 380
   Leu Leu Arg Gln Thr Ala Gly Lys Val Leu Ala His Phe Tyr Gly Val
   385                 390                 395                 400
   Lys Leu Glu Gly Lys Val Pro Met His Lys Leu Phe Leu Glu Met Leu
                   405                 410                 415
   Glu Ala Met Met Asp
               420
<210> 16
   <211> 511
   <212> PRT
   <213> Homo sapiens
<400> 16
   Met Ser Arg Gly Pro Glu Glu Val Asn Arg Leu Thr Glu Ser Thr Tyr
   1               5                   10                  15
   Arg Asn Val Met Glu Gln Phe Asn Pro Gly Leu Arg Asn Leu Ile Asn
               20                  25                  30
   Leu Gly Lys Asn Tyr Glu Lys Ala Val Asn Ala Met Ile Leu Ala Gly
           35                  40                  45
   Lys Ala Tyr Tyr Asp Gly Val Ala Lys Ile Gly Glu Ile Ala Thr Gly
       50                  55                  60
   Ser Pro Val Ser Thr Glu Leu Gly His Val Leu Ile Glu Ile Ser Ser
   65                  70                  75                  80
   Thr His Lys Lys Leu Asn Glu Ser Leu Asp Glu Asn Phe Lys Lys Phe
                   85                  90                  95
   His Lys Glu Ile Ile His Glu Leu Glu Lys Lys Ile Glu Leu Asp Val
               100                 105                 110
   Lys Tyr Met Asn Ala Thr Leu Lys Arg Tyr Gln Thr Glu His Lys Asn
           115                 120                 125
   Lys Leu Glu Ser Leu Glu Lys Ser Gln Ala Glu Leu Lys Lys Ile Arg
       130                 135                 140
   Arg Lys Ser Gln Gly Ser Arg Asn Ala Leu Lys Tyr Glu His Lys Glu
   145                 150                 155                 160
   Ile Glu Tyr Val Glu Thr Val Thr Ser Arg Gln Ser Glu Ile Gln Lys
                   165                 170                 175
   Phe Ile Ala Asp Gly Cys Lys Glu Ala Leu Leu Glu Glu Lys Arg Arg
               180                 185                 190
   Phe Cys Phe Leu Val Asp Lys His Cys Gly Phe Ala Asn His Ile His
           195                 200                 205
   Tyr Tyr His Leu Gln Ser Ala Glu Leu Leu Asn Ser Lys Leu Pro Arg
       210                 215                 220
   Trp Gln Glu Thr Cys Val Asp Ala Ile Lys Val Pro Glu Lys Ile Met
   225                 230                 235                 240
   Asn Met Ile Glu Glu Ile Lys Thr Pro Ala Ser Thr Pro Val Ser Gly
                   245                 250                 255
   Thr Pro Gln Ala Ser Pro Met Ile Glu Arg Ser Asn Val Val Arg Lys
               260                 265                 270
   Asp Tyr Asp Thr Leu Ser Lys Cys Ser Pro Lys Met Pro Pro Ala Pro
           275                 280                 285
   Ser Gly Arg Ala Tyr Thr Ser Pro Leu Ile Asp Met Phe Asn Asn Pro
       290                 295                 300
   Ala Thr Ala Ala Pro Asn Ser Gln Arg Val Asn Asn Ser Thr Gly Thr
   305                 310                 315                 320
   Ser Glu Asp Pro Ser Leu Gln Arg Ser Val Ser Val Ala Thr Gly Leu
                   325                 330                 335
   Asn Met Met Lys Lys Gln Lys Val Lys Thr Ile Phe Pro His Thr Ala
               340                 345                 350
   Gly Ser Asn Lys Thr Leu Leu Ser Phe Ala Gln Gly Asp Val Ile Thr
           355                 360                 365
   Leu Leu Ile Pro Glu Glu Lys Asp Gly Trp Leu Tyr Gly Glu His Asp
       370                 375                 380
   Val Ser Lys Ala Arg Gly Trp Phe Pro Ser Ser Tyr Thr Lys Leu Leu
   385                 390                 395                 400
   Glu Glu Asn Glu Thr Glu Ala Val Thr Val Pro Thr Pro Ser Pro Thr
                   405                 410                 415
   Pro Val Arg Ser Ile Ser Thr Val Asn Leu Ser Glu Asn Ser Ser Val
               420                 425                 430
   Val Ile Pro Pro Pro Asp Tyr Leu Glu Cys Leu Ser Met Gly Ala Ala
           435                 440                 445
   Ala Asp Arg Arg Ala Asp Ser Ala Arg Thr Thr Ser Thr Phe Lys Ala
       450                 455                 460
   Pro Ala Ser Lys Pro Glu Thr Ala Ala Pro Asn Asp Ala Asn Gly Thr
   465                 470                 475                 480
   Ala Lys Pro Pro Phe Leu Ser Gly Glu Asn Pro Phe Ala Thr Val Lys
                   485                 490                 495
   Leu Arg Pro Thr Val Thr Asn Asp Arg Ser Ala Pro Ile Ile Arg
               500                 505                 510
<210> 17
   <211> 124
   <212> PRT
   <213> Homo sapiens
<400> 17
   Met Ala Ala Ala Met Thr Phe Cys Arg Leu Leu Asn Arg Cys Gly Glu
   1               5                   10                  15
   Ala Ala Arg Ser Leu Pro Leu Gly Ala Arg Cys Phe Gly Val Arg Val
               20                  25                  30
   Ser Pro Thr Gly Glu Lys Val Thr His Thr Gly Gln Val Tyr Asp Asp
           35                  40                  45
   Lys Asp Tyr Arg Arg Ile Arg Phe Val Gly Arg Gln Lys Glu Val Asn
       50                  55                  60
   Glu Asn Phe Ala Ile Asp Leu Ile Ala Glu Gln Pro Val Ser Glu Val
   65                  70                  75                  80
   Glu Thr Arg Val Ile Ala Cys Asp Gly Gly Gly Gly Ala Leu Gly His
                   85                  90                  95
   Pro Lys Val Tyr Ile Asn Leu Asp Lys Glu Thr Lys Thr Gly Thr Cys
               100                 105                 110
   Gly Tyr Cys Gly Leu Gln Phe Arg Gln His His His
           115                 120
<210> 18
   <211> 206
   <212> PRT
   <213> Homo sapiens
<400> 18
<210> 19
   <211> 411
   <212> PRT
   <213> Homo sapiens
<400> 19
   Met Ile Pro Ser Arg His Thr Met Leu Arg Phe Leu Pro Val Val Asn
   1 5 10 15
   Ala Ala Ser Cys Pro Gly Asp Arg Arg Thr Met Leu Val Asn Val Ala
   20 25 30
   Ala Gly Val Arg Val Leu Arg Val Pro Leu Arg Ala Gly Gly Ser Gly
   35 40 45
   Ser Leu Ser Gly Leu Arg Pro Pro Ala Ile Val Cys Tyr Leu Pro Leu
   50 55 60
   Gln Arg Ala Ser Ala Ala Ser Gly Leu Phe Leu Ala Arg Pro Gln His
   65 70 75 80
   Cys Gly Arg Cys Gly Arg Gly Arg Gly Gly Ala Ala Leu Ser Leu Gly
   85 90 95
   Ser Pro Ala Tyr Ala Ser Arg Cys Arg Val Ser Arg Ala Ala Val Phe
   100 105 110
   Ser Pro Trp Ala Pro Val Ser Leu Glu Ser Gly Arg Ala Pro Gly Cys
   115 120 125
   Ser Leu Gly Arg Pro Gly Leu Arg Gly Ala Leu Val Val Trp Leu Gln
   130 135 140
   Leu Gly Glu Thr Trp Val Arg Leu Arg Gly Asp Phe Gln Pro Ala Cys
   145 150 155 160
   Gly Val Val Arg Val Glu Arg Leu Ala Gly Tyr Arg Asp Ala Gly His
   165 170 175
   Gln Gly Leu Asp Gly Ala Gly Pro Ala Val Trp Val Leu Arg Asp Val
                   180                 185                 190
   Ala Gln Val Pro Ala Asp Arg Ser Ala Tyr Cys Gly Ala Ser Leu Ala
               195                 200                 205
   Gly Pro Arg Gly Ala Leu Ser Leu Leu Leu Leu Leu Leu Ala Pro Pro
       210                 215                 220
   Ser Arg Pro Ala Ala Gly Cys Pro Ala Pro Cys Ser Cys Ala Gly Thr
   225                 230                 235                 240
   Leu Val Asp Cys Gly Arg Arg Gly Leu Thr Trp Ala Ser Leu Pro Thr
                   245                 250                 255
   Ala Phe Pro Val Asp Thr Thr Glu Leu Val Leu Thr Gly Asn Asn Leu
               260                 265                 270
   Thr Ala Leu Pro Pro Gly Leu Leu Asp Ala Leu Pro Ala Leu Arg Thr
           275                 280                 285
   Ala His Leu Gly Ala Asn Pro Trp Arg Cys Asp Cys Arg Leu Val Pro
       290                 295                 300
   Leu Arg Ala Trp Leu Ala Gly Arg Pro Glu Arg Ala Pro Tyr Arg Asp
   305                 310                 315                 320
   Leu Arg Cys Val Ala Pro Pro Ala Leu Arg Gly Arg Leu Leu Pro Tyr
                   325                 330                 335
   Leu Ala Glu Asp Glu Leu Arg Ala Ala Cys Ala Pro Gly Pro Leu Cys
               340                 345                 350
   Trp Gly Ala Leu Ala Ala Gln Leu Ala Leu Leu Gly Leu Gly Leu Leu
           355                 360                 365
   His Ala Leu Leu Leu Val Leu Leu Leu Cys Arg Leu Arg Arg Leu Arg
       370                 375                 380
   Ala Arg Ala Arg Ala Arg Ala Ala Ala Arg Leu Ser Leu Thr Asp Pro
   385                 390                 395                 400
   Leu Val Ala Glu Arg Ala Gly Thr Asp Glu Ser
                   405                 410
<210> 20
   <211> 916
   <212> PRT
   <213> Homo sapiens
<400> 20
   Met Val Gln Lys Glu Ser Gln Ala Thr Leu Glu Glu Arg Glu Ser Glu
   1               5                   10                  15
   Leu Ser Ser Asn Pro Ala Ala Ser Ala Gly Ala Ser Leu Glu Pro Pro
               20                  25                  30
   Ala Ala Pro Ala Pro Gly Glu Asp Asn Pro Ala Gly Ala Gly Gly Ala
           35                  40                  45
   Ala Val Ala Gly Ala Ala Gly Gly Ala Arg Arg Phe Leu Cys Gly Val
       50                  55                  60
   Val Glu Gly Phe Tyr Gly Arg Pro Trp Val Met Glu Gln Arg Lys Glu
   65                  70                  75                  80
   Leu Phe Arg Arg Leu Gln Lys Trp Glu Leu Asn Thr Tyr Leu Tyr Ala
                   85                  90                  95
   Pro Lys Asp Asp Tyr Lys His Arg Met Phe Trp Arg Glu Met Tyr Ser
               100                 105                 110
   Val Glu Glu Ala Glu Gln Leu Met Thr Leu Ile Ser Ala Ala Arg Glu
           115                 120                 125
   Tyr Glu Ile Glu Phe Ile Tyr Ala Ile Ser Pro Gly Leu Asp Ile Thr
       130                 135                 140
   Phe Ser Asn Pro Lys Glu Val Ser Thr Leu Lys Arg Lys Leu Asp Gln
   145                 150                 155                 160
   Val Ser Gln Phe Gly Cys Arg Ser Phe Ala Leu Leu Phe Asp Asp Ile
                   165                 170                 175
   Asp His Asn Met Cys Ala Ala Asp Lys Glu Val Phe Ser Ser Phe Ala
               180                 185                 190
   His Ala Gln Val Ser Ile Thr Asn Glu Ile Tyr Gln Tyr Leu Gly Glu
           195                 200                 205
   Pro Glu Thr Phe Leu Phe Cys Pro Thr Glu Tyr Cys Gly Thr Phe Cys
       210                 215                 220
   Tyr Pro Asn Val Ser Gln Ser Pro Tyr Leu Arg Thr Val Gly Glu Lys
   225                 230                 235                 240
   Leu Leu Pro Gly Ile Glu Val Leu Trp Thr Gly Pro Lys Val Val Ser
                   245                 250                 255
   Lys Glu Ile Pro Val Glu Ser Ile Glu Glu Val Ser Lys Ile Ile Lys
               260                 265                 270
   Arg Ala Pro Val Ile Trp Asp Asn Ile His Ala Asn Asp Tyr Asp Gln
           275                 280                 285
   Lys Arg Leu Phe Leu Gly Pro Tyr Lys Gly Arg Ser Thr Glu Leu Ile
       290                 295                 300
   Pro Arg Leu Lys Gly Val Leu Thr Asn Pro Asn Cys Glu Phe Glu Ala
   305                 310                 315                 320
   Asn Tyr Val Ala Ile His Thr Leu Ala Thr Trp Tyr Lys Ser Asn Met
                   325                 330                 335
   Asn Gly Val Arg Lys Asp Val Val Met Thr Asp Ser Glu Asp Ser Thr
               340                 345                 350
   Val Ser Ile Gln Ile Lys Leu Glu Asn Glu Gly Ser Asp Glu Asp Ile
           355                 360                 365
   Glu Thr Asp Val Leu Tyr Ser Pro Gln Met Ala Leu Lys Leu Ala Leu
       370                 375                 380
   Thr Glu Trp Leu Gln Glu Phe Gly Val Pro His Gln Tyr Ser Ser Arg
   385                 390                 395                 400
   Gln Val Ala His Ser Gly Ala Lys Ala Ser Val Val Asp Gly Thr Pro
                   405                 410                 415
   Leu Val Ala Ala Pro Ser Leu Asn Ala Thr Thr Val Val Thr Thr Val
               420                 425                 430
   Tyr Gln Glu Pro Ile Met Ser Gln Gly Ala Ala Leu Ser Gly Glu Pro
           435                 440                 445
   Thr Thr Leu Thr Lys Glu Glu Glu Lys Lys Gln Pro Asp Glu Glu Pro
       450                 455                 460
   Met Asp Met Val Val Glu Lys Gln Glu Glu Thr Asp His Lys Asn Asp
   465                 470                 475                 480
   Asn Gln Ile Leu Ser Glu Ile Val Glu Ala Lys Met Ala Glu Glu Leu
   485 490 495
   Lys Pro Met Asp Thr Asp Lys Glu Ser Ile Ala Glu Ser Lys Ser Pro
   500 505 510
   Glu Met Ser Met Gln Glu Asp Cys Ile Ser Asp Ile Ala Pro Met Gln
   515 520 525
   Thr Asp Glu Gln Thr Asn Lys Glu Gln Phe Val Pro Gly Pro Asn Glu
   530 535 540
   Lys Pro Leu Tyr Thr Ala Glu Pro Val Thr Leu Glu Asp Leu Gln Leu
   545 550 555 560
   Leu Ala Asp Leu Phe Tyr Leu Pro Tyr Glu His Gly Pro Lys Gly Ala
   565 570 575
   Gln Met Leu Arg Glu Phe Gln Trp Leu Arg Ala Asn Ser Ser Val Val
   580 585 590
   Ser Val Asn Cys Lys Gly Lys Asp Ser Glu Lys Ile Glu Glu Trp Arg
   595 600 605
   Ser Arg Ala Ala Lys Phe Glu Glu Met Cys Gly Leu Val Met Gly Met
   610 615 620
   Phe Thr Arg Leu Ser Asn Cys Ala Asn Arg Thr Ile Leu Tyr Asp Met
   625 630 635 640
   Tyr Ser Tyr Val Trp Asp Ile Lys Ser Ile Met Ser Met Val Lys Ser
   645 650 655
   Phe Val Gln Trp Leu Gly Cys Arg Ser His Ser Ser Ala Gln Phe Leu
   660 665 670
   Ile Gly Asp Gln Glu Pro Trp Ala Phe Arg Gly Gly Leu Ala Gly Glu
   675 680 685
   Phe Gln Arg Leu Leu Pro Ile Asp Gly Ala Asn Asp Leu Phe Phe Gln
   690 695 700
   Pro Pro Pro Leu Thr Pro Thr Ser Lys Val Tyr Thr Ile Arg Pro Tyr
   705 710 715 720
   Phe Pro Lys Asp Glu Ala Ser Val Tyr Lys Ile Cys Arg Glu Met Tyr
   725 730 735
   Asp Asp Gly Val Gly Leu Pro Phe Gln Ser Gln Pro Asp Leu Ile Gly
   740 745 750
   Asp Lys Leu Val Gly Gly Leu Leu Ser Leu Ser Leu Asp Tyr Cys Phe
   755 760 765
   Val Leu Glu Asp Glu Asp Gly Ile Cys Gly Tyr Ala Leu Gly Thr Val
   770 775 780
   Asp Val Thr Pro Phe Ile Lys Lys Cys Lys Ile Ser Trp Ile Pro Phe
   785 790 795 800
   Met Gln Glu Lys Tyr Thr Lys Pro Asn Gly Asp Lys Glu Leu Ser Glu
   805 810 815
   Ala Glu Lys Ile Met Leu Ser Phe His Glu Glu Gln Glu Val Leu Pro
   820 825 830
   Glu Thr Phe Leu Ala Asn Phe Pro Ser Leu Ile Lys Met Asp Ile His
   835 840 845
   Lys Lys Val Thr Asp Pro Ser Val Ala Lys Ser Met Met Ala Cys Leu
   850 855 860
   Leu Ser Ser Leu Lys Ala Asn Gly Ser Arg Gly Ala Phe Cys Glu Val
   865 870 875 880
   Arg Pro Asp Asp Lys Arg Ile Leu Glu Phe Tyr Ser Lys Leu Gly Cys
   885 890 895
   Phe Glu Ile Ala Lys Met Glu Gly Phe Pro Lys Asp Val Val Ile Leu
   900 905 910
   Gly Arg Ser Leu
   915
<210> 21
   <211> 849
   <212> PRT
   <213> Homo sapiens
<400> 21
   Met Val Gln Lys Glu Ser Gln Ala Thr Leu Glu Glu Arg Glu Ser Glu
   1 5 10 15
   Leu Ser Ser Asn Pro Ala Ala Ser Ala Gly Ala Ser Leu Glu Pro Pro
   20 25 30
   Ala Ala Pro Ala Pro Gly Glu Asp Asn Pro Ala Gly Ala Gly Gly Ala
           35                  40                  45
   Ala Val Ala Gly Ala Ala Gly Gly Ala Arg Arg Phe Leu Cys Gly Val
       50                  55                  60
   Val Glu Gly Phe Tyr Gly Arg Pro Trp Val Met Glu Gln Arg Lys Glu
   65                  70                  75                  80
   Leu Phe Arg Arg Leu Gln Lys Trp Glu Leu Asn Thr Tyr Leu Tyr Ala
                   85                  90                  95
   Pro Lys Asp Asp Tyr Lys His Arg Met Phe Trp Arg Glu Met Tyr Ser
               100                 105                 110
   Val Glu Glu Ala Glu Gln Leu Met Thr Leu Ile Ser Ala Ala Arg Glu
           115                 120                 125
   Tyr Glu Ile Glu Phe Ile Tyr Ala Ile Ser Pro Gly Leu Asp Ile Thr
       130                 135                 140
   Phe Ser Asn Pro Lys Glu Val Ser Thr Leu Lys Arg Lys Leu Asp Gln
   145                 150                 155                 160
   Val Ser Gln Phe Gly Cys Arg Ser Phe Ala Leu Leu Phe Asp Asp Ile
                   165                 170                 175
   Asp His Asn Met Cys Ala Ala Asp Lys Glu Val Phe Ser Ser Phe Ala
               180                 185                 190
   His Ala Gln Val Ser Ile Thr Asn Glu Ile Tyr Gln Tyr Leu Gly Glu
           195                 200                 205
   Pro Glu Thr Phe Leu Phe Cys Pro Thr Glu Tyr Cys Gly Thr Phe Cys
       210                 215                 220
   Tyr Pro Asn Val Ser Gln Ser Pro Tyr Leu Arg Thr Val Gly Glu Lys
   225                 230                 235                 240
   Leu Leu Pro Gly Ile Glu Val Leu Trp Thr Gly Pro Lys Val Val Ser
                   245                 250                 255
   Lys Glu Ile Pro Val Glu Ser Ile Glu Glu Val Ser Lys Ile Ile Lys
               260                 265                 270
   Arg Ala Pro Val Ile Trp Asp Asn Ile His Ala Asn Asp Tyr Asp Gln
           275                 280                 285
   Lys Arg Leu Phe Leu Gly Pro Tyr Lys Gly Arg Ser Thr Glu Leu Ile
       290                 295                 300
   Pro Arg Leu Lys Gly Val Leu Thr Asn Pro Asn Cys Glu Phe Glu Ala
   305                 310                 315                 320
   Asn Tyr Val Ala Ile His Thr Leu Ala Thr Trp Tyr Lys Ser Asn Met
                   325                 330                 335
   Asn Gly Val Arg Lys Asp Val Val Met Ser Arg Gln Val Ala His Ser
               340                 345                 350
   Gly Ala Lys Ala Ser Val Val Asp Gly Thr Pro Leu Val Ala Ala Pro
           355                 360                 365
   Ser Leu Asn Ala Thr Thr Val Val Thr Thr Val Tyr Gln Glu Pro Ile
       370                 375                 380
   Met Ser Gln Gly Ala Ala Leu Ser Gly Glu Pro Thr Thr Leu Thr Lys
   385                 390                 395                 400
   Glu Glu Glu Lys Lys Gln Pro Asp Glu Glu Pro Met Asp Met Val Val
                   405                 410                 415
   Glu Lys Gln Glu Glu Thr Asp His Lys Asn Asp Asn Gln Ile Leu Ser
               420                 425                 430
   Glu Ile Val Glu Ala Lys Met Ala Glu Glu Leu Lys Pro Met Asp Thr
           435                 440                 445
   Asp Lys Glu Ser Ile Ala Glu Ser Lys Ser Pro Glu Met Ser Met Gln
       450                 455                 460
   Glu Asp Cys Ile Ser Asp Ile Ala Pro Met Gln Thr Asp Glu Gln Thr
   465                 470                 475                 480
   Asn Lys Glu Gln Phe Val Pro Gly Pro Asn Glu Lys Pro Leu Tyr Thr
                   485                 490                 495
   Ala Glu Pro Val Thr Leu Glu Asp Leu Gln Leu Leu Ala Asp Leu Phe
               500                 505                 510
   Tyr Leu Pro Tyr Glu His Gly Pro Lys Gly Ala Gln Met Leu Arg Glu
           515                 520                 525
   Phe Gln Trp Leu Arg Ala Asn Ser Ser Val Val Ser Val Asn Cys Lys
       530                 535                 540
   Gly Lys Asp Ser Glu Lys Ile Glu Glu Trp Arg Ser Arg Ala Ala Lys
   545                 550                 555                 560
   Phe Glu Glu Met Cys Gly Leu Val Met Gly Met Phe Thr Arg Leu Ser
                   565                 570                 575
   Asn Cys Ala Asn Arg Thr Ile Leu Tyr Asp Met Tyr Ser Tyr Val Trp
               580                 585                 590
   Asp Ile Lys Ser Ile Met Ser Met Val Lys Ser Phe Val Gln Trp Leu
           595                 600                 605
   Gly Cys Arg Ser His Ser Ser Ala Gln Phe Leu Ile Gly Asp Gln Glu
       610                 615                 620
   Pro Trp Ala Phe Arg Gly Gly Leu Ala Gly Glu Phe Gln Pro Pro Pro
   625                 630                 635                 640
   Leu Thr Pro Thr Ser Lys Val Tyr Thr Ile Arg Pro Tyr Phe Pro Lys
                   645                 650                 655
   Asp Glu Ala Ser Val Tyr Lys Ile Cys Arg Glu Met Tyr Asp Asp Gly
               660                 665                 670
   Val Gly Leu Pro Phe Gln Ser Gln Pro Asp Leu Ile Gly Asp Lys Leu
           675                 680                 685
   Val Gly Gly Leu Leu Ser Leu Ser Leu Asp Tyr Cys Phe Val Leu Glu
       690                 695                 700
   Asp Glu Asp Gly Ile Cys Gly Tyr Ala Leu Gly Thr Val Asp Val Thr
   705                 710                 715                 720
   Pro Phe Ile Lys Lys Cys Lys Ile Ser Trp Ile Pro Phe Met Gln Glu
                   725                 730                 735
   Lys Tyr Thr Lys Pro Asn Gly Asp Lys Glu Leu Ser Glu Ala Glu Lys
               740                 745                 750
   Ile Met Leu Ser Phe His Glu Glu Gln Glu Val Leu Pro Glu Thr Phe
           755                 760                 765
   Leu Ala Asn Phe Pro Ser Leu Ile Lys Met Asp Ile His Lys Lys Val
       770                 775                 780
   Thr Asp Pro Ser Val Ala Lys Ser Met Met Ala Cys Leu Leu Ser Ser
   785 790 795 800
   Leu Lys Ala Asn Gly Ser Arg Gly Ala Phe Cys Glu Val Arg Pro Asp
   805 810 815
   Asp Lys Arg Ile Leu Glu Phe Tyr Ser Lys Leu Gly Cys Phe Glu Ile
   820 825 830
   Ala Lys Met Glu Gly Phe Pro Lys Asp Val Val Ile Leu Gly Arg Ser
   835 840 845
   Leu
<210> 22
   <211> 676
   <212> PRT
   <213> Homo sapiens
<400> 22
   Met Val Gln Lys Glu Ser Gln Ala Thr Leu Glu Glu Arg Glu Ser Glu
   1 5 10 15
   Leu Ser Ser Asn Pro Ala Ala Ser Ala Gly Ala Ser Leu Glu Pro Pro
   20 25 30
   Ala Ala Pro Ala Pro Gly Glu Asp Asn Pro Ala Gly Ala Gly Gly Ala
   35 40 45
   Ala Val Ala Gly Ala Ala Gly Gly Ala Arg Arg Phe Leu Cys Gly Val
   50 55 60
   Val Glu Gly Phe Tyr Gly Arg Pro Trp Val Met Glu Gln Arg Lys Glu
   65 70 75 80
   Leu Phe Arg Arg Leu Gln Lys Trp Glu Leu Asn Thr Tyr Leu Tyr Ala
   85 90 95
   Pro Lys Asp Asp Tyr Lys His Arg Met Phe Trp Arg Glu Met Tyr Ser
   100 105 110
   Val Glu Glu Ala Glu Gln Leu Met Thr Leu Ile Ser Ala Ala Arg Glu
   115 120 125
   Tyr Glu Ile Glu Phe Ile Tyr Ala Ile Ser Pro Gly Leu Asp Ile Thr
   130 135 140
   Phe Ser Asn Pro Lys Glu Val Ser Thr Leu Lys Arg Lys Leu Asp Gln
   145 150 155 160
   Val Ser Gln Phe Gly Cys Arg Ser Phe Ala Leu Leu Phe Asp Asp Ile
   165 170 175
   Asp His Asn Met Cys Ala Ala Asp Lys Glu Val Phe Ser Ser Phe Ala
   180 185 190
   His Ala Gln Val Ser Ile Thr Asn Glu Ile Tyr Gln Tyr Leu Gly Glu
   195 200 205
   Pro Glu Thr Phe Leu Phe Cys Pro Thr Glu Tyr Cys Gly Thr Phe Cys
   210 215 220
   Tyr Pro Asn Val Ser Gln Ser Pro Tyr Leu Arg Thr Val Gly Glu Lys
   225 230 235 240
   Leu Leu Pro Gly Ile Glu Val Leu Trp Thr Gly Pro Lys Val Val Ser
   245 250 255
   Lys Glu Ile Pro Val Glu Ser Ile Glu Glu Val Ser Lys Ile Ile Lys
   260 265 270
   Arg Ala Pro Val Ile Trp Asp Asn Ile His Ala Asn Asp Tyr Asp Gln
   275 280 285
   Lys Arg Leu Phe Leu Gly Pro Tyr Lys Gly Arg Ser Thr Glu Leu Ile
   290 295 300
   Pro Arg Leu Lys Gly Val Leu Thr Asn Pro Asn Cys Glu Phe Glu Ala
   305 310 315 320
   Asn Tyr Val Ala Ile His Thr Leu Ala Thr Trp Tyr Lys Ser Asn Met
   325 330 335
   Asn Gly Val Arg Lys Asp Val Val Met Thr Asp Ser Glu Asp Ser Thr
   340 345 350
   Val Ser Ile Gln Ile Lys Leu Glu Asn Glu Gly Ser Asp Glu Asp Ile
   355 360 365
   Glu Thr Asp Val Leu Tyr Ser Pro Gln Met Ala Leu Lys Leu Ala Leu
   370 375 380
   Thr Glu Trp Leu Gln Glu Phe Gly Val Pro His Gln Tyr Ser Ser Arg
   385 390 395 400
   Gln Val Ala His Ser Gly Ala Lys Ala Ser Val Val Asp Gly Thr Pro
                   405                 410                 415
   Leu Val Ala Ala Pro Ser Leu Asn Ala Thr Thr Val Val Thr Thr Val
               420                 425                 430
   Tyr Gln Glu Pro Ile Met Ser Gln Gly Ala Ala Leu Ser Gly Glu Pro
           435                 440                 445
   Thr Thr Leu Thr Lys Glu Glu Glu Lys Lys Gln Pro Asp Glu Glu Pro
       450                 455                 460
   Met Asp Met Val Val Glu Lys Gln Glu Glu Thr Asp His Lys Asn Asp
   465                 470                 475                 480
   Asn Gln Ile Leu Ser Glu Ile Val Glu Ala Lys Met Ala Glu Glu Leu
                   485                 490                 495
   Lys Pro Met Asp Thr Asp Lys Glu Ser Ile Ala Glu Ser Lys Ser Pro
               500                 505                 510
   Glu Met Ser Met Gln Glu Asp Cys Ile Ser Asp Ile Ala Pro Met Gln
           515                 520                 525
   Thr Asp Glu Gln Thr Asn Lys Glu Gln Phe Val Pro Gly Pro Asn Glu
       530                 535                 540
   Lys Pro Leu Tyr Thr Ala Glu Pro Val Thr Leu Glu Asp Leu Gln Leu
   545                 550                 555                 560
   Leu Ala Asp Leu Phe Tyr Leu Pro Tyr Glu His Gly Pro Lys Gly Ala
                   565                 570                 575
   Gln Met Leu Arg Glu Phe Gln Trp Leu Arg Ala Asn Ser Ser Val Val
               580                 585                 590
   Ser Val Asn Cys Lys Gly Lys Asp Ser Glu Lys Ile Glu Glu Trp Arg
           595                 600                 605
   Ser Arg Ala Ala Lys Phe Glu Glu Met Cys Gly Leu Val Met Gly Met
       610                 615                 620
   Phe Thr Arg Leu Ser Asn Cys Ala Asn Arg Thr Ile Leu Tyr Asp Met
   625                 630                 635                 640
   Tyr Ser Tyr Val Trp Asp Ile Lys Ser Ile Met Ser Met Val Lys Ser
                   645                 650                 655
   Phe Val Gln Trp Leu Gly Arg Cys Thr Arg Asn Asn Leu Phe Ser Ser
   660 665 670
   Asn Ile Leu Ser
   675
<210> 23
   <211> 863
   <212> PRT
   <213> Homo sapiens
<400> 23
   Met Val Gln Lys Glu Ser Gln Ala Thr Leu Glu Glu Arg Glu Ser Glu
   1 5 10 15
   Leu Ser Ser Asn Pro Ala Ala Ser Ala Gly Ala Ser Leu Glu Pro Pro
   20 25 30
   Ala Ala Pro Ala Pro Gly Glu Asp Asn Pro Ala Gly Ala Gly Gly Ala
   35 40 45
   Ala Val Ala Gly Ala Ala Gly Gly Ala Arg Arg Phe Leu Cys Gly Val
   50 55 60
   Val Glu Gly Phe Tyr Gly Arg Pro Trp Val Met Glu Gln Arg Lys Glu
   65 70 75 80
   Leu Phe Arg Arg Leu Gln Lys Trp Glu Leu Asn Thr Tyr Leu Tyr Ala
   85 90 95
   Pro Lys Asp Asp Tyr Lys His Arg Met Phe Trp Arg Glu Met Tyr Ser
   100 105 110
   Val Glu Glu Ala Glu Gln Leu Met Thr Leu Ile Ser Ala Ala Arg Glu
   115 120 125
   Tyr Glu Ile Glu Phe Ile Tyr Ala Ile Ser Pro Gly Leu Asp Ile Thr
   130 135 140
   Phe Ser Asn Pro Lys Glu Val Ser Thr Leu Lys Arg Lys Leu Asp Gln
   145 150 155 160
   Val Ser Gln Phe Gly Cys Arg Ser Phe Ala Leu Leu Phe Asp Asp Ile
   165 170 175
   Asp His Asn Met Cys Ala Ala Asp Lys Glu Val Phe Ser Ser Phe Ala
   180 185 190
   His Ala Gln Val Ser Ile Thr Asn Glu Ile Tyr Gln Tyr Leu Gly Glu
           195                 200                 205
   Pro Glu Thr Phe Leu Phe Cys Pro Thr Glu Tyr Cys Gly Thr Phe Cys
       210                 215                 220
   Tyr Pro Asn Val Ser Gln Ser Pro Tyr Leu Arg Thr Val Gly Glu Lys
   225                 230                 235                 240
   Leu Leu Pro Gly Ile Glu Val Leu Trp Thr Gly Pro Lys Val Val Ser
                   245                 250                 255
   Lys Glu Ile Pro Val Glu Ser Ile Glu Glu Val Ser Lys Ile Ile Lys
               260                 265                 270
   Arg Ala Pro Val Ile Trp Asp Asn Ile His Ala Asn Asp Tyr Asp Gln
           275                 280                 285
   Lys Arg Leu Phe Leu Gly Pro Tyr Lys Gly Arg Ser Thr Glu Leu Ile
       290                 295                 300
   Pro Arg Leu Lys Gly Val Leu Thr Asn Pro Asn Cys Glu Phe Glu Ala
   305                 310                 315                 320
   Asn Tyr Val Ala Ile His Thr Leu Ala Thr Trp Tyr Lys Ser Asn Met
                   325                 330                 335
   Asn Gly Val Arg Lys Asp Val Val Met Ser Arg Gln Val Ala His Ser
               340                 345                 350
   Gly Ala Lys Ala Ser Val Val Asp Gly Thr Pro Leu Val Ala Ala Pro
           355                 360                 365
   Ser Leu Asn Ala Thr Thr Val Val Thr Thr Val Tyr Gln Glu Pro Ile
       370                 375                 380
   Met Ser Gln Gly Ala Ala Leu Ser Gly Glu Pro Thr Thr Leu Thr Lys
   385                 390                 395                 400
   Glu Glu Glu Lys Lys Gln Pro Asp Glu Glu Pro Met Asp Met Val Val
                   405                 410                 415
   Glu Lys Gln Glu Glu Thr Asp His Lys Asn Asp Asn Gln Ile Leu Ser
               420                 425                 430
   Glu Ile Val Glu Ala Lys Met Ala Glu Glu Leu Lys Pro Met Asp Thr
           435                 440                 445
   Asp Lys Glu Ser Ile Ala Glu Ser Lys Ser Pro Glu Met Ser Met Gln
   450 455 460
   Glu Asp Cys Ile Ser Asp Ile Ala Pro Met Gln Thr Asp Glu Gln Thr
   465 470 475 480
   Asn Lys Glu Gln Phe Val Pro Gly Pro Asn Glu Lys Pro Leu Tyr Thr
   485 490 495
   Ala Glu Pro Val Thr Leu Glu Asp Leu Gln Leu Leu Ala Asp Leu Phe
   500 505 510
   Tyr Leu Pro Tyr Glu His Gly Pro Lys Gly Ala Gln Met Leu Arg Glu
   515 520 525
   Phe Gln Trp Leu Arg Ala Asn Ser Ser Val Val Ser Val Asn Cys Lys
   530 535 540
   Gly Lys Asp Ser Glu Lys Ile Glu Glu Trp Arg Ser Arg Ala Ala Lys
   545 550 555 560
   Phe Glu Glu Met Cys Gly Leu Val Met Gly Met Phe Thr Arg Leu Ser
   565 570 575
   Asn Cys Ala Asn Arg Thr Ile Leu Tyr Asp Met Tyr Ser Tyr Val Trp
   580 585 590
   Asp Ile Lys Ser Ile Met Ser Met Val Lys Ser Phe Val Gln Trp Leu
   595 600 605
   Gly Cys Arg Ser His Ser Ser Ala Gln Phe Leu Ile Gly Asp Gln Glu
   610 615 620
   Pro Trp Ala Phe Arg Gly Gly Leu Ala Gly Glu Phe Gln Arg Leu Leu
   625 630 635 640
   Pro Ile Asp Gly Ala Asn Asp Leu Phe Phe Gln Pro Pro Pro Leu Thr
   645 650 655
   Pro Thr Ser Lys Val Tyr Thr Ile Arg Pro Tyr Phe Pro Lys Asp Glu
   660 665 670
   Ala Ser Val Tyr Lys Ile Cys Arg Glu Met Tyr Asp Asp Gly Val Gly
   675 680 685
   Leu Pro Phe Gln Ser Gln Pro Asp Leu Ile Gly Asp Lys Leu Val Gly
       690                 695                 700
   Gly Leu Leu Ser Leu Ser Leu Asp Tyr Cys Phe Val Leu Glu Asp Glu
   705                 710                 715                 720
   Asp Gly Ile Cys Gly Tyr Ala Leu Gly Thr Val Asp Val Thr Pro Phe
                   725                 730                 735
   Ile Lys Lys Cys Lys Ile Ser Trp Ile Pro Phe Met Gln Glu Lys Tyr
               740                 745                 750
   Thr Lys Pro Asn Gly Asp Lys Glu Leu Ser Glu Ala Glu Lys Ile Met
           755                 760                 765
   Leu Ser Phe His Glu Glu Gln Glu Val Leu Pro Glu Thr Phe Leu Ala
       770                 775                 780
   Asn Phe Pro Ser Leu Ile Lys Met Asp Ile His Lys Lys Val Thr Asp
   785                 790                 795                 800
   Pro Ser Val Ala Lys Ser Met Met Ala Cys Leu Leu Ser Ser Leu Lys
                   805                 810                 815
   Ala Asn Gly Ser Arg Gly Ala Phe Cys Glu Val Arg Pro Asp Asp Lys
               820                 825                 830
   Arg Ile Leu Glu Phe Tyr Ser Lys Leu Gly Cys Phe Glu Ile Ala Lys
           835                 840                 845
   Met Glu Gly Phe Pro Lys Asp Val Val Ile Leu Gly Arg Ser Leu
       850                 855                 860
<210> 24
   <211> 364
   <212> PRT
   <213> Homo sapiens
<400> 24
<210> 25
   <211> 132
   <212> PRT
   <213> Homo sapiens
<400> 25
   Ala Val Leu Val Thr Gly Cys Asp Ser Gly Phe Gly Phe Ser Leu Ala
   1 5 10 15
   Lys His Leu His Ser Lys Gly Phe Leu Val Phe Ala Gly Cys Leu Leu
   20 25 30
   Lys Glu Val Ala Glu Val Asn Leu Trp Gly Thr Val Arg Ser Phe Leu
   35 40 45
   Pro Leu Leu Arg Arg Val Val Asn Ile Ser Ser Met Leu Gly Arg Ser
   50 55 60
   Pro Tyr Cys Ile Thr Lys Phe Gly Val Glu Ala Phe Ser Asp Cys Leu
   65 70 75 80
   Arg Tyr Glu Met His Pro Leu Gly Val Lys Val Ser Val Val Glu Pro
   85 90 95
   Gly Asn Phe Ile Ala Ala Thr Ser Leu Tyr Ser Pro Glu Arg Met Trp
   100 105 110
   Asp Glu Leu Pro Glu Val Val Arg Lys Tyr His Pro Met Asp Tyr Tyr
   115 120 125
   Trp Trp Leu Arg
   130
<210> 26
   <211> 256
   <212> PRT
   <213> Homo sapiens
<400> 26
   Met Leu Ser Arg Val Val Leu Ser Ala Ala Ala Thr Ala Ala Pro Ser
   1               5                   10                  15
   Leu Lys Asn Ala Ala Phe Leu Gly Pro Gly Val Leu Gln Ala Thr Arg
               20                  25                  30
   Thr Phe His Thr Gly Gln Pro His Leu Val Pro Val Pro Pro Leu Pro
           35                  40                  45
   Glu Tyr Gly Gly Lys Val Arg Tyr Gly Leu Ile Pro Glu Glu Phe Phe
       50                  55                  60
   Gln Phe Leu Tyr Pro Lys Thr Gly Val Thr Gly Pro Tyr Val Leu Gly
   65                  70                  75                  80
   Thr Gly Leu Ile Leu Tyr Ala Leu Ser Lys Glu Ile Tyr Val Ile Ser
                   85                  90                  95
   Ala Glu Thr Phe Thr Ala Leu Ser Val Leu Gly Val Met Val Tyr Gly
               100                 105                 110
   Ile Lys Lys Tyr Gly Pro Phe Val Ala Asp Phe Ala Asp Lys Leu Asn
           115                 120                 125
   Glu Gln Lys Leu Ala Gln Leu Glu Glu Ala Lys Gln Ala Ser Ile Gln
       130                 135                 140
   His Ile Gln Asn Ala Ile Asp Thr Glu Lys Ser Gln Gln Ala Leu Val
   145                 150                 155                 160
   Gln Lys Arg His Tyr Leu Phe Asp Val Gln Arg Asn Asn Ile Ala Met
                   165                 170                 175
   Ala Leu Glu Val Thr Tyr Arg Glu Arg Leu Tyr Arg Val Tyr Lys Glu
               180                 185                 190
   Val Lys Asn Arg Leu Asp Tyr His Ile Ser Val Gln Asn Met Met Arg
           195                 200                 205
   Arg Lys Glu Gln Glu His Met Ile Asn Trp Val Glu Lys His Val Val
       210                 215                 220
   Gln Ser Ile Ser Thr Gln Gln Glu Lys Glu Thr Ile Ala Lys Cys Ile
   225                 230                 235                 240
   Ala Asp Leu Lys Leu Leu Ala Lys Lys Ala Gln Ala Gln Pro Val Met
                   245                 250                 255
<210> 27
   <211> 406
   <212> PRT
   <213> Homo sapiens
<400> 27
   Met Pro Pro Val Gly Gly Lys Lys Ala Lys Lys Gly Ile Leu Glu Arg
   1               5                   10                  15
   Leu Asn Ala Gly Glu Ile Val Ile Gly Asp Gly Gly Phe Val Phe Ala
               20                  25                  30
   Leu Glu Lys Arg Gly Tyr Val Lys Ala Gly Pro Trp Thr Pro Glu Ala
           35                  40                  45
   Ala Val Glu His Pro Glu Ala Val Arg Gln Leu His Arg Glu Phe Leu
       50                  55                  60
   Arg Ala Gly Ser Asn Val Met Gln Thr Phe Thr Phe Tyr Ala Ser Glu
   65                  70                  75                  80
   Asp Lys Leu Glu Asn Arg Gly Asn Tyr Val Leu Glu Lys Ile Ser Gly
                   85                  90                  95
   Gln Glu Val Asn Glu Ala Ala Cys Asp Ile Ala Arg Gln Val Ala Asp
               100                 105                 110
   Glu Gly Asp Ala Leu Val Ala Gly Gly Val Ser Gln Thr Pro Ser Tyr
           115                 120                 125
   Leu Ser Cys Lys Ser Glu Thr Glu Val Lys Lys Val Phe Leu Gln Gln
       130                 135                 140
   Leu Glu Val Phe Met Lys Lys Asn Val Asp Phe Leu Ile Ala Glu Tyr
   145                 150                 155                 160
   Phe Glu His Val Glu Glu Ala Val Trp Ala Val Glu Thr Leu Ile Ala
                   165                 170                 175
   Ser Gly Lys Pro Val Ala Ala Thr Met Cys Ile Gly Pro Glu Gly Asp
               180                 185                 190
   Leu His Gly Val Pro Pro Gly Glu Cys Ala Val Arg Leu Val Lys Ala
           195                 200                 205
   Gly Ala Ser Ile Ile Gly Val Asn Cys His Phe Asp Pro Thr Ile Ser
   210 215 220
   Leu Lys Thr Val Lys Leu Met Lys Glu Gly Leu Glu Ala Ala Arg Leu
   225 230 235 240
   Lys Ala His Leu Met Ser Gln Pro Leu Ala Tyr His Thr Pro Asp Cys
   245 250 255
   Asn Lys Gln Gly Phe Ile Asp Leu Pro Glu Phe Pro Phe Gly Leu Glu
   260 265 270
   Pro Arg Val Ala Thr Arg Trp Asp Ile Gln Lys Tyr Ala Arg Glu Ala
   275 280 285
   Tyr Asn Leu Gly Val Arg Tyr Ile Gly Gly Cys Cys Gly Phe Glu Pro
   290 295 300
   Tyr His Ile Arg Ala Ile Ala Glu Glu Leu Ala Pro Glu Arg Gly Phe
   305 310 315 320
   Leu Pro Pro Ala Ser Glu Lys His Gly Ser Trp Gly Ser Gly Leu Asp
   325 330 335
   Met His Thr Lys Pro Trp Val Arg Ala Arg Ala Arg Lys Glu Tyr Trp
   340 345 350
   Glu Asn Leu Arg Ile Ala Ser Gly Arg Pro Tyr Asn Pro Ser Met Ser
   355 360 365
   Lys Pro Asp Gly Trp Gly Val Thr Lys Gly Thr Ala Glu Leu Met Gln
   370 375 380
   Gln Lys Glu Ala Thr Thr Glu Gln Gln Leu Lys Glu Leu Phe Glu Lys
   385 390 395 400
   Gln Lys Phe Lys Ser Gln
   405
<210> 28
   <211> 454
   <212> PRT
   <213> Homo sapiens
<400> 28
   Met Cys Ala Ala Gln Met Pro Pro Leu Ala His Ile Phe Arg Gly Thr
   1 5 10 15
   Phe Val His Ser Thr Trp Thr Cys Pro Met Glu Val Leu Arg Asp His
               20                  25                  30
   Leu Leu Gly Val Ser Asp Ser Gly Lys Ile Val Phe Leu Glu Glu Ala
           35                  40                  45
   Ser Gln Gln Glu Lys Leu Ala Lys Glu Trp Cys Phe Lys Pro Cys Glu
       50                  55                  60
   Ile Arg Glu Leu Ser His His Glu Phe Phe Met Pro Gly Leu Val Asp
   65                  70                  75                  80
   Thr His Ile His Ala Ser Gln Tyr Ser Phe Ala Gly Ser Ser Ile Asp
                   85                  90                  95
   Leu Pro Leu Leu Glu Trp Leu Thr Lys Tyr Thr Phe Pro Ala Glu His
               100                 105                 110
   Arg Phe Gln Asn Ile Asp Phe Ala Glu Glu Val Tyr Thr Arg Val Val
           115                 120                 125
   Arg Arg Thr Leu Lys Asn Gly Thr Thr Thr Ala Cys Tyr Phe Ala Thr
       130                 135                 140
   Ile His Thr Asp Ser Ser Leu Leu Leu Ala Asp Ile Thr Asp Lys Phe
   145                 150                 155                 160
   Gly Gln Arg Ala Phe Val Gly Lys Val Cys Met Asp Leu Asn Asp Thr
                   165                 170                 175
   Phe Pro Glu Tyr Lys Glu Thr Thr Glu Glu Ser Ile Lys Glu Thr Glu
               180                 185                 190
   Arg Phe Val Ser Glu Met Leu Gln Lys Asn Tyr Ser Arg Val Lys Pro
           195                 200                 205
   Ile Val Thr Pro Arg Phe Ser Leu Ser Cys Ser Glu Thr Leu Met Gly
       210                 215                 220
   Glu Leu Gly Asn Ile Ala Lys Thr Arg Asp Leu His Ile Gln Ser His
   225                 230                 235                 240
   Ile Ser Glu Asn Arg Asp Glu Val Glu Ala Val Lys Asn Leu Tyr Pro
                   245                 250                 255
   Ser Tyr Lys Asn Tyr Thr Ser Val Tyr Asp Lys Asn Asn Leu Leu Thr
               260                 265                 270
   Asn Lys Thr Val Met Ala His Gly Cys Tyr Leu Ser Ala Glu Glu Leu
           275                 280                 285
   Asn Val Phe His Glu Arg Gly Ala Ser Ile Ala His Cys Pro Asn Ser
       290                 295                 300
   Asn Leu Ser Leu Ser Ser Gly Phe Leu Asn Val Leu Glu Val Leu Lys
   305                 310                 315                 320
   His Glu Val Lys Ile Gly Leu Gly Thr Asp Val Ala Gly Gly Tyr Ser
                   325                 330                 335
   Tyr Ser Met Leu Asp Ala Ile Arg Arg Ala Val Met Val Ser Asn Ile
               340                 345                 350
   Leu Leu Ile Asn Lys Val Asn Glu Lys Ser Leu Thr Leu Lys Glu Val
           355                 360                 365
   Phe Arg Leu Ala Thr Leu Gly Gly Ser Gln Ala Leu Gly Leu Asp Gly
       370                 375                 380
   Glu Ile Gly Asn Phe Glu Val Gly Lys Glu Phe Asp Ala Ile Leu Ile
   385                 390                 395                 400
   Asn Pro Lys Ala Ser Asp Ser Pro Ile Asp Leu Phe Tyr Gly Asp Phe
                   405                 410                 415
   Phe Gly Asp Ile Ser Glu Ala Val Ile Gln Lys Phe Leu Tyr Leu Gly
               420                 425                 430
   Asp Asp Arg Asn Ile Glu Glu Val Tyr Val Gly Gly Lys Gln Val Val
           435                 440                 445
   Pro Phe Ser Ser Ser Val
       450
<210> 29
   <211> 380
   <212> PRT
   <213> Homo sapiens
<400> 29
   Met Pro Gly Leu Val Asp Thr His Ile His Ala Ser Gln Tyr Ser Phe
   1               5                   10                  15
   Ala Gly Ser Ser Ile Asp Leu Pro Leu Leu Glu Trp Leu Thr Lys Tyr
               20                  25                  30
   Thr Phe Pro Ala Glu His Arg Phe Gln Asn Ile Asp Phe Ala Glu Glu
           35                  40                  45
   Val Tyr Thr Arg Val Val Arg Arg Thr Leu Lys Asn Gly Thr Thr Thr
       50                  55                  60
   Ala Cys Tyr Phe Ala Thr Ile His Thr Asp Ser Ser Leu Leu Leu Ala
   65                  70                  75                  80
   Asp Ile Thr Asp Lys Phe Gly Gln Arg Ala Phe Val Gly Lys Val Cys
                   85                  90                  95
   Met Asp Leu Asn Asp Thr Phe Pro Glu Tyr Lys Glu Thr Thr Glu Glu
               100                 105                 110
   Ser Ile Lys Glu Thr Glu Arg Phe Val Ser Glu Met Leu Gln Lys Asn
           115                 120                 125
   Tyr Ser Arg Val Lys Pro Ile Val Thr Pro Arg Phe Ser Leu Ser Cys
       130                 135                 140
   Ser Glu Thr Leu Met Gly Glu Leu Gly Asn Ile Ala Lys Thr Arg Asp
   145                 150                 155                 160
   Leu His Ile Gln Ser His Ile Ser Glu Asn Arg Asp Glu Val Glu Ala
                   165                 170                 175
   Val Lys Asn Leu Tyr Pro Ser Tyr Lys Asn Tyr Thr Ser Val Tyr Asp
               180                 185                 190
   Lys Asn Asn Leu Leu Thr Asn Lys Thr Val Met Ala His Gly Cys Tyr
           195                 200                 205
   Leu Ser Ala Glu Glu Leu Asn Val Phe His Glu Arg Gly Ala Ser Ile
       210                 215                 220
   Ala His Cys Pro Asn Ser Asn Leu Ser Leu Ser Ser Gly Phe Leu Asn
   225                 230                 235                 240
   Val Leu Glu Val Leu Lys His Glu Val Lys Ile Gly Leu Gly Thr Asp
                   245                 250                 255
   Val Ala Gly Gly Tyr Ser Tyr Ser Met Leu Asp Ala Ile Arg Arg Ala
               260                 265                 270
   Val Met Val Ser Asn Ile Leu Leu Ile Asn Lys Val Asn Glu Lys Ser
           275                 280                 285
   Leu Thr Leu Lys Glu Val Phe Arg Leu Ala Thr Leu Gly Gly Ser Gln
   290 295 300
   Ala Leu Gly Leu Asp Gly Glu Ile Gly Asn Phe Glu Val Gly Lys Glu
   305 310 315 320
   Phe Asp Ala Ile Leu Ile Asn Pro Lys Ala Ser Asp Ser Pro Ile Asp
   325 330 335
   Leu Phe Tyr Gly Asp Phe Phe Gly Asp Ile Ser Glu Ala Val Ile Gln
   340 345 350
   Lys Phe Leu Tyr Leu Gly Asp Asp Arg Asn Ile Glu Glu Val Tyr Val
   355 360 365
   Gly Gly Lys Gln Val Val Pro Phe Ser Ser Ser Val
   370 375 380
<210> 30
   <211> 471
   <212> PRT
   <213> Homo sapiens
<400> 30
   Met Cys Ala Ala Gln Met Pro Pro Leu Ala His Ile Phe Arg Gly Thr
   1 5 10 15
   Phe Val His Ser Thr Trp Thr Cys Pro Met Glu Val Leu Arg Asp His
   20 25 30
   Leu Leu Gly Val Ser Asp Ser Gly Lys Ile Val Phe Leu Glu Glu Ala
   35 40 45
   Ser Gln Gln Glu Lys Leu Ala Lys Glu Trp Cys Phe Lys Pro Cys Glu
   50 55 60
   Ile Arg Glu Leu Ser His His Glu Phe Phe Met Pro Gly Leu Val Asp
   65 70 75 80
   Thr His Ile His Ala Ser Gln Tyr Ser Phe Ala Gly Ser Ser Ile Asp
   85 90 95
   Leu Pro Leu Leu Glu Trp Leu Thr Lys Tyr Thr Phe Pro Ala Glu His
   100 105 110
   Arg Phe Gln Asn Ile Asp Phe Ala Glu Glu Val Tyr Thr Arg Val Val
   115 120 125
   Arg Arg Thr Leu Lys Asn Gly Thr Thr Thr Ala Cys Tyr Phe Ala Thr
       130                 135                 140
   Ile His Thr Asp Ser Ser Leu Leu Leu Ala Asp Ile Thr Asp Lys Phe
   145                 150                 155                 160
   Gly Gln Arg Ala Phe Val Gly Lys Val Cys Met Asp Leu Asn Asp Thr
                   165                 170                 175
   Phe Pro Glu Tyr Lys Glu Thr Thr Glu Glu Ser Ile Lys Glu Thr Glu
               180                 185                 190
   Arg Phe Val Ser Glu Met Leu Gln Lys Asn Tyr Ser Arg Val Lys Pro
           195                 200                 205
   Ile Val Thr Pro Arg Phe Ser Leu Ser Cys Ser Glu Thr Leu Met Gly
       210                 215                 220
   Glu Leu Gly Asn Ile Ala Lys Thr Arg Asp Leu His Ile Gln Ser His
   225                 230                 235                 240
   Ile Ser Glu Asn Arg Asp Glu Val Glu Ala Val Lys Asn Leu Tyr Pro
                   245                 250                 255
   Ser Tyr Lys Asn Tyr Thr Ser Val Tyr Asp Lys Asn Asn Leu Leu Thr
               260                 265                 270
   Asn Lys Thr Val Met Ala His Gly Cys Tyr Leu Ser Ala Glu Glu Leu
           275                 280                 285
   Asn Val Phe His Glu Arg Gly Ala Ser Ile Ala His Cys Pro Asn Ser
       290                 295                 300
   Asn Leu Ser Leu Ser Ser Gly Phe Leu Asn Val Leu Glu Val Leu Lys
   305                 310                 315                 320
   His Glu Val Lys Ile Gly Leu Gly Thr Asp Val Ala Gly Gly Tyr Ser
                   325                 330                 335
   Tyr Ser Met Leu Asp Ala Ile Arg Arg Ala Val Met Val Ser Asn Ile
               340                 345                 350
   Leu Leu Ile Asn Lys Val Asn Glu Lys Ser Leu Thr Leu Lys Glu Val
           355                 360                 365
   Phe Arg Leu Ala Thr Leu Gly Gly Ser Gln Ala Leu Gly Leu Asp Gly
               370                 375                 380
   Glu Ile Gly Asn Phe Glu Val Gly Lys Glu Phe Asp Ala Ile Leu Ile
   385                 390                 395                 400
   Asn Pro Lys Ala Ser Asp Ser Pro Ile Asp Leu Phe Tyr Gly Asp Phe
                   405                 410                 415
   Phe Gly Asp Ile Ser Glu Ala Val Ile Gln Lys Phe Leu Tyr Leu Gly
               420                 425                 430
   Asp Asp Arg Asn Ile Glu Glu Val Tyr Val Gly Gly Lys Gln Val Val
           435                 440                 445
   Pro Phe Ser Ser Ser Val Lys Glu Thr Ile His Leu Pro Ala Ser Ser
       450                 455                 460
   Pro His Pro Pro Pro Phe Pro
   465                 470
<210> 31
   <211> 201
   <212> PRT
   <213> Homo sapiens
<400> 31
   Met Ala Leu Ser Trp Val Leu Thr Val Leu Ser Leu Leu Pro Leu Leu
   1               5                   10                  15
   Glu Ala Gln Ile Pro Leu Cys Ala Asn Leu Val Pro Val Pro Ile Thr
               20                  25                  30
   Asn Ala Thr Leu Asp Gln Ile Thr Gly Lys Trp Phe Tyr Ile Ala Ser
           35                  40                  45
   Ala Phe Arg Asn Glu Glu Tyr Asn Lys Ser Val Gln Glu Ile Gln Ala
       50                  55                  60
   Thr Phe Phe Tyr Phe Thr Pro Asn Lys Thr Glu Asp Thr Ile Phe Leu
   65                  70                  75                      80
   Arg Glu Tyr Gln Thr Arg Gln Asp Gln Cys Ile Tyr Asn Thr Thr Tyr
                   85                  90                  95
   Leu Asn Val Gln Arg Glu Asn Gly Thr Ile Ser Arg Tyr Val Gly Gly
               100                 105                 110
   Gln Glu His Phe Ala His Leu Leu Ile Leu Arg Asp Thr Lys Thr Tyr
           115                 120                 125
   Met Leu Ala Phe Asp Val Asn Asp Glu Lys Asn Trp Gly Leu Ser Val
       130                 135                 140
   Tyr Ala Asp Lys Pro Glu Thr Thr Lys Glu Gln Leu Gly Glu Phe Tyr
   145                 150                 155                     160
   Glu Ala Leu Asp Cys Leu Arg Ile Pro Lys Ser Asp Val Val Tyr Thr
                   165                 170                 175
   Asp Trp Lys Lys Asp Lys Cys Glu Pro Leu Glu Lys Gln His Glu Lys
               180                 185                 190
   Glu Arg Lys Gln Glu Glu Gly Glu Ser
           195                 200

## Description

### Technical Field of the Invention

The present invention is related to a method for *in vitro* diagnosis of diabetes, obesity or metabolic diseases associated thereto, with the use of at least one protein molecule as a biomarker selected from the group of E3 ubiquitin-protein ligase RNF181, UPF0361 protein C3orf37 homolog, inhibin beta B chain, or a DNA or mRNA thereof, or a RNA molecule which is selected from miRNAs and IncRNAs that target mRNAs thereof.

### Background of the Invention

Diabetes is a chronic disease which is characterized by almost complete death of the beta cell amount (type 1) or diminishing of a certain part thereof (type 2). Therefore, beta cell mass regulation represents a critical issue for understanding of the disease. However, this mechanism is still not fully understood. Innate beta cells of individuals originate by the proliferation and differentiation (neogenesis) of pancreatic progenitor cells. After birth, beta cell neogenesis ends and increased insulin requirement is met by the proliferation beta cells with an ability to divide in few numbers. Mature beta cells with a low-level proliferation capacity do not enable a significant regeneration against advanced tissue damage. Therefore, beta cells which fail to meet the metabolic requirements that are increased chronically, were damaged. At this point, progenitor cells which are found in islet internally or externally, may play an important role in increasing beta cell mass. However, these cells require to be stimulated to differentiate. For therapeutic purposes, it should be considered that stem cells which locate especially in pancreatic islets may also differentiate into beta cells.

Keeping blood glucose at a certain level is regulated with cells located in Langerhans islet which compose the endocrine part of pancreas. Islet cells secrete hormone to normalize high or low levels by following blood glucose concentration. Following food intake when blood glucose is increased, beta cells decrease blood glucose by secreting insulin. Insulin stimulates blood glucose intake by insulin sensitive cells of the body and glucose is converted into glycogen in liver. In other words, regulation of blood glucose level is reached by the rapid change in insulin synthesis and secretion which takes place in pancreatic beta cells. This is directly related with beta cell mass. Beta cell replication regulated by glucose hormonal effect, apoptotic elimination and neogenesis originated from progenitor cells in special conditions are factors that affect beta cell mass. Changes such as distinctions in cell mass, pregnancy, sensitivity of peripheral tissues for insulin or tissue damage may cause glucose to increase chronically and diabetes in advanced levels.

Global increase in diabetes makes it mandatory to develop novel therapeutic strategies such as increasing beta cell amount or regenerative medicine. Applications of exogenous insulin or hypoglycemic agent which are among the applied therapies for type 1-2 diabetes, are not satisfactory because they are not therapeutic and are inadequate to prevent the generation of secondary complications related to diabetes. However, because of the amount of beta cells obtained from cadaver liver and immunologic problems, transplantable patient number is limited. This problem can only be overcome by increasing the amount of beta cells obtained from donors. As an alternative approach for cell transplantation, increasing endogen beta cell mass in patient's pancreas by growth factor applications can be contemplated. In order to apply both of the applications the mechanism which regulates pancreatic beta cell mass should be identified.

It is known that the regeneration capacity of beta cells is quite low without a suitable stimulant. This situation is probably is the result of the limited replication capacity of beta cells and the generation of beta cells from precursor cells by neogenesis is not continuously active. However, when a suitable potent external stimulant is applied a regenerative cell increase may occur. This type of regenerative growth can be originated from the present precursor/progenitor or stem cell.

Calorigenic foods, particularly glucose and esterified lipid acids (NEFA) are essential regulators of pancreatic beta cell function. Application of glucose in high concentration in short term to beta cells, increases secretion and biosynthesis of insulin. However, this situation also causes degeneration of beta cell function and apoptosis (glucotoxicity) which is characterized by chronic hyperglycemia and decrease of insulin biosynthesis. Similarly, acute NEFA application also increases insulin secretion stimulated by glucose from beta cells, however the long-term application of NEFA in high concentration triggers beta cell apoptosis (lipotoxicity).

Researchers formed "glucolipotoxicity" condition by applying glucose together with NEFA at high concentration and demonstrated that this application has a synergistic lethal effect on beta cells. Glucolipotoxicity may have an important role in decompensation phase which occurs during obesity related Type 2 diabetes development. It was showed that addition of NEFA and triglyceride at high levels to hyperglycemia which occurs after food intake and continuous hyperglycemia increases beta cell apoptosis and destructs beta cell function.

According to increased amount of data, it is known that beta cells or at least beta cell like cells can be generated from differentiated stem cell by *in vitro* methods. Although the scientific meaning of these findings are not known completely, the potential to use these differentiatable cells as a source to regain beta cell mass is present

Stem cells are identified as clonogenic, self-regenerate, multipotent cells and found in many organs such as bone marrow, bowel, skin, brain. It is suggested that exocrine glands and islets can be progenitor cell source in postnatal pancreas. Although this possibility was suggested long time ago, only a few articles support the presence of stem cells or progenitor cells found in adult pancreas. One of the published articles asserts that these cells can be isolated from normal rodent pancreas and be subcultered during long terms. Moreover, it is noted that in *in vitro* differentiation conditions these self-regenerative cells can differentiate into cells that can produce insulin and when transplanted they can cure diabetes. It is not known whether this progenitor or stem cells have a role in *in vivo* beta cell regeneration, increase in amount or regeneration. However, it shouldn't be ignored that this type of cells are located in pancreas and can be used in producing beta cells *in vitro.*

It was determined that development of diabetes takes place in five consecutive steps. Every step is characterized by significant change for beta cell mass, phenotype and function. The most well-defined step is the first step and named as compensation. In this step, insulin secretion increases to keep normal glucose levels as to this type of change is an indicator of obesity, physical inactivity and insulin resistance induced by genetic tendency. In the second step, fasting glucose levels increases to values of 5-6.5 mmol/L (89-116 mg/dL) that indicates a consistent beta cell adaptation. Third step is a variable period of early decompensation, in fourth step which comes after this step increased glucose levels are consistent (decompensation). In the fifth step which is the final step, advanced decompensation is characterized by beta cell insufficiency and ketos generation.

The most important indicator of compensation which is the first step of diabetes development is the significant increase in glucose stimulated insulin secretion. The cause of this insulin secretion increase is definitely the increase in the amount of pancreatic β cell. Normally the number of pancreatic β cells increases or decreases with the change of the equilibrium between the cell generated by cellular division or differentiation and the number of the lost β cell by way of apoptotic cell death. The cause of the increase in the number of pancreatic β cell occurring in the first step of diabetes development and the source of increased β cell are not fully understood. The answer of this question is very important for the prevention of hyperglycemia by way of increasing pancreatic β cells which are suggested to play a central role in the development of diabetes. As mentioned above in detail, one of the potential cell sources that are able to give β cells in pancreas is pancreatic mesenchymal stem cells.

In state of art, there have been studies about the use of different proteins in treatment of diabetes, obesity and associated metabolic diseases thereto and generally cell death and cell differentiation related diseases. The use of distinct molecules is crucial because diseases that are targeted for diagnosis are highly variable and the disease inherently had a complicated structure.

In the scope of the invention it was aimed to develop novel methods for diagnosis of said diseases by using particular molecules set forth below.

### Brief Description of the Invention

The present invention makes use of the below molecules for the diagnosis of diabetes, obesity or metabolic diseases associated thereto:
- E3 ubiquitin-protein ligase RNF181,
- UPF0361 protein C3orf37 homolog,
- Inhibin beta B chain,
or DNA, mRNA thereof or an RNA molecule which is selected from miRNAs and IncRNAs targeting mRNAs thereof.

The said disease may be diabetes or obesity. Said diabetes can be Type I or Type II diabetes.

The metabolic diseases associated to diabetes and obesity disclosed herein are preferably selected from lactose intolerance, fructose intolerance, galactosemia, glycogen storage disease, insulin resistance syndrome, syndrome X, retinopathy, neuropathy, nephropathy, foot ulcers, hypertension, hyperlipidemia, metabolic syndrome, osteoarthritis, cardiovascular diseases, paralysis, sleep apnea, liver disease, asthma, respiration difficulty, menstruation disorders, musculoskeletal disorders, dermatologic disorders, polycystic ovary syndrome, immune system disorders.

More particularly, the invention is related to a method for *in vitro* diagnosis of diabetes, obesity or metabolic diseases associated thereto, the method comprises use of at least one protein molecule as a biomarker selected from the group of:
- E3 ubiquitin-protein ligase RNF181,
- UPF0361 protein C3orf37 homolog,
- Inhibin beta B chain,
or a DNA or mRNA thereof, or an RNA molecule which is selected from miRNAs and
IncRNAs that target mRNAs thereof.

The disease which is diagnosed by this method is preferably Type I or Type II diabetes. The method can be carried out by detecting the presence of said biomarkers in a patient's blood, plasma, serum, milk, bronchoalveolar fluid or cerebrospinal fluid. The method preferably comprises the detection of the said molecules by way of ELISA, liquid chromatography, mass spectrometry, western blotting, southern blotting, northern blotting, PCR, RT-PCR, qRT-PCR or microarray assay.

### Objects of the Invention

The object of the invention is to present novel biomarkers that can be used for *in vitro* diagnosis of diabetes, obesity and metabolic diseases associated thereto in the early stage of the progress of the diseases.

### Brief Description of the Figures

Figures explaining the invention are appended to the description. Brief description of the figures is given hereinbelow.
**Figure 1** - Morphology of pancreatic islets and mesenchymal cells stemmed therefrom (A) The islet isolated from rats, is dyed with dithizone; an insulin marker. (B) Viability of islets isolated from rats was determined by marking with h fluorescein diacetate (green) and propidium iodide (red). Almost all of the islets were recorded as viable. After 1 and 3 days in culture medium, growing body of fibroblast-like cells (arrow) which are separated from islets, were observed. Original magnifications: (A, C, D) x 200; (B) x 100, I: Pancreatic islet.
**Figure 2** - Analysis results of flow cytometry that show passage pancreatic islet originated stem cells specific cell surface markers.
**Figure 3** - Analysis results of Western blotting that show passage pancreatic islet originated stem cells specific intracellular markers. ENG: Endoglin; NES: Nestin; VIM: Vimentin; FN: Fibronectin.
**Figure 4** - Graph shows the amount of viable pancreatic islet originated stem cells on 1^{th}, 4^{th}, 7^{th}, 11^{th} and 14^{th} days. The amount of viable cells was calculated by WST1 test and absorbance values were measured as 450 nm wavelength. In the forthcoming days, absorbance values that show the amount of cell viability, were recorded to be significantly increased.
   ^{a}p<0.001 vs. 1^{th} day, ^{b}p<0.001 vs. 4^{th} day, ^{c}p<0.001 vs. 7^{th}, ^{d}p<0.001 vs.11^{th} day Absorbance values: 0.14±0.01 on 1^{th} day, 0.54±0.03 on 4^{th} day, 0.65±0.09 on 7^{th} day, 01.19±0.04 on 11^{th} day, 1.99±0.12 on 14^{th} day. Results are given as mean ± SEM.

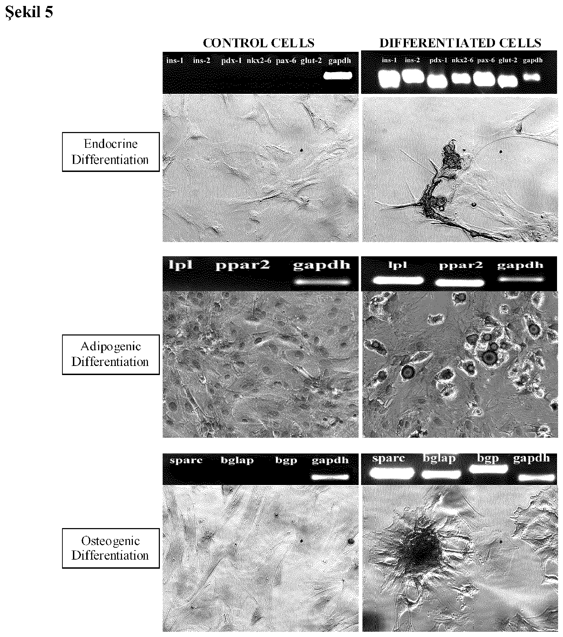

**Figure 5** - RT-PCR products and photomicrographs related to *in vitro* endocrine, adipogenic and osteogenic differentiation assay for pancreatic islet originated mesenchymal stem cells. On upper panel, endocrine differentiation was shown by the demonstration of pancreatic β-cell specific molecules: ins-1, ins-2, pdx-1, nkx2-6, pax-6, and glut-2's RT-PCR products and by marking insulin positive β-cells immunohistochemically. On middle panel, adipogenic differentiation was shown by demonstration of adipocyte specific molecules: lpl and ppar2's RT-PCR products and by marking oil vacuoles histochemically with oil red O. On bottom panel, osteogenic differentiation was shown by the demonstration of osteocyte specific molecules: sparc, bglap and bgp's RT-PCR products and by marking mineral nodule vacuoles histochemically with Alizarin red S. Original magnification: x 200.
**Figure 6** - (A) Graph shows apoptosis rate change of β-cells, pancreatic islet originated stem cells, β-cells taken from β-cells+PI-MSCs coculture system and PI-MSCs taken from β-cells+PI-MSCs coculture system when glucotoxicity, lipotoxicity and glucolipotoxicity conditions applied. *p<0.05, **p<0.01, ***p<0.001. (B) Table shows apoptosis rates as multiple increase. Results are given as mean ± SEM.
**Figure 7** - Graph shows necrosis rate change which is calculated according to LDH secretion of β-cells, pancreatic islet originated stem cells, β-cells taken from β-cells+PI-MSCs coculture system and PI-MSCs taken from β-cells+PI-MSCs coculture system under glucotoxicity, lipotoxicity and glucolipotoxicity conditions.
   *p<0.05, **p<0.01, ***p<0.001. (B) Table shows necrosis rates as secreted LDH (mU/mg protein) values. Results are given as mean ± SEM.
**Figure 8** - Graph shows proliferation rate change which is calculated according to BrdU marking of β-cells and β-cells taken from β-cells+PI-MSCs coculture system under glucotoxicity, lipotoxicity and glucolipotoxicity conditions.
   ***p<0.001, ^{#}p<0.05, ^{###}p<0.001, ^{ψ}p<0.05. (B) Table shows proliferation rates as multiple increase. Results are given as mean ± SEM.

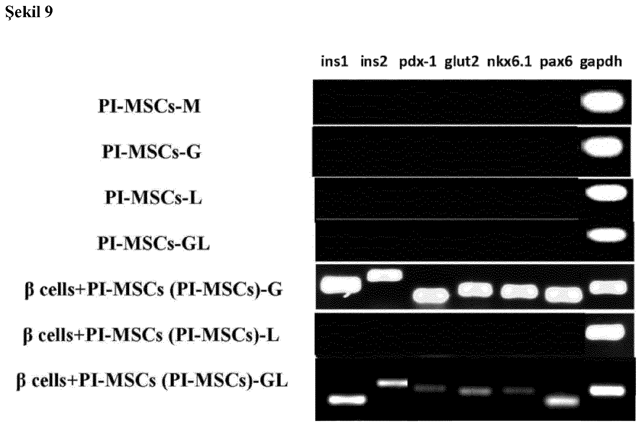

**Figure 9** - (A) RT-PCR analysis results for PI-MSCs taken from β-cells+PI-MSCs coculture system under glucotoxicity, lipotoxicity and glucolipotoxicity conditions.
**Figure 10** - (A) Increases glucose stimulated insulin secretion results of β-cells, pancreatic islet originated stem cells, β-cells taken from β-cells+PI-MSCs coculture system and PI-MSCs taken from β-cells+PI-MSCs coculture system.
   ***p<0001 every single experiment condition according to 5.5 mmol/L of Glucose value, ^{#}p<0.05 and ^{###}p<0.001 every single experiment condition after application of medium according to applied 5.5 mmol/L of Glucose value. M: FBS ve BSA including medium; G: Glucotoxicity, L: Lipotoxicity ve GL: Glucolipotoxicity. (B) Table shows insulin levels (ng/mg protein). Results are given as mean ± SEM.

**Table 1** - Peptides determined by mass spectrometry on samples obtained from spots where differentiation observed after 2D electrophoresis.

### Disclosure of the Invention

The invention is related to a method for *in vitro* diagnosis of diabetes, obesity or metabolic diseases associated thereto, wherein the method comprises the use of at least one protein molecule selected from following group as a biomarker:
- E3 ubiquitin-protein ligase RNF181,
- UPF0361 protein C3orf37 homolog,
- Inhibin beta B chain,
or a DNA or mRNA thereof, or a molecule which is selected from miRNAs and IncRNAs that target mRNAs thereof.

Within the scope of the *in vitro* diagnosis method of the invention, ELISA, mass spectrometry, blotting or other proteomic techniques which enable the measurement of specific proteins in low concentrations in all body fluids such as blood, plasma, serum, milk, bronchoalveolar fluid or cerebrospinal fluid, can be used. Said *in vitro* diagnosis method may involve detection by sensible methods such as for example southern blotting, northern blotting, PCR, RT-PCR, qRT-PCR, microarray assay and sequencing. Diabetes according to the invention may be Type I or Type II diabetes.

In preferred embodiments, the said disease is diabetes, wherein said molecules can be screened *in vitro* as biomarkers in serum, plasma and other body fluids of pre-diabetic or diabetic individuals. Screening of these in body fluids can be conducted by protein measurement methods such as ELISA or enzyme activity assays that can be carried out colorimetric techniques or techniques based on hybridization such as PCR, qRT-PCR, microarray assay and sequencing.

The term "mRNA" used in the present invention refers to messenger RNA. mRNA molecules convey genetic information from DNA to ribosome. The terms "mRNA" and "messenger RNA" used in the scope of the present invention have the same meaning and can be used interchangeably.

The term "miRNA" used in the present invention refers to RNA molecule that is small and non-coding. These molecules can be used in RNA silencing and gene expression regulation after transcription. The terms "micro RNA" and "miRNA" have the same meaning and can be used interchangeably.

The term "lncRNA" used in the present invention refers to long non-coding RNAs. These RNAs are non-protein coding transcripts and have a size more than 200 nucleotides.

In the scope of the invention the terms "lncRNA" and "long non-coding RNA" have the same meaning and can be used interchangeably. The term "diabetes" used in the present invention means type I diabetes and/or type II diabetes.

"Diabetes and obesity associated metabolic diseases" used in the present invention comprises lactose intolerance, fructose intolerance, galactosemia, glycogen storage disease, insulin resistance syndrome, syndrome X, retinopathy, neuropathy, nephropathy, foot ulcers, hypertension, hyperlipidemia, metabolic syndrome, gallbladder diseases, osteoarthritis, cardiovascular diseases, paralysis, sleep apnea, liver disease, asthma, respiration difficulty, menstruation disorders, musculoskeletal disorders, dermatologic disorders, polycystic ovary syndrome and immune system disorders.

The molecule named "E3 ubiquitin-protein ligase RNF181" used herein comprises a peptide consisting of 153 amino acids, derivatives of this molecule known in prior art and synonyms thereof. Said derivatives may be those formed by chemical modification of the E3 ubiquitin-protein ligase RNF181 or by metabolism of the E3 ubiquitin-protein ligase RNF181. Amino acid sequence of E3 ubiquitin-protein ligase RNF181 is: MASYFDEHDCEPSDPEQETRTNMLLELARSLFNRMDFEDLGLVVDWDHHLP PPAAKTVVENLPRTVIRGSQAELKCPVCLLEFEEEETAIEMPCHHLFHSSCILP WLSKTNSCPLCRYELPTDDDTYEEHRRDKARKQQQQHRLENLHGAMYT (SEQ ID NO:1), wherein its weight is 17,909 Da. The E3 ubiquitin-protein ligase RNF181, ubiquitin from the E2 ubiquitin-conjugated enzyme, takes ubiquitin and transfers it to the target protein. It is associated with cell proliferation. In embryonic stem cells, E3 ubiquitin-protein ligases binding to Trim25 and Trim71 RNA were found to fall with differentiation (Kwon et al., 2013). It has also been shown that E3 ubiquitin-protein ligase is required for the differentiation of follicular helper T cells (Xiao et al., 2014).

In this context, all of the given data relating to the use of the E3 ubiquitin-protein ligase RNF181 in the context of the present invention include derivatives and synonyms of this molecule. In other words, all derivatives and synonyms of the E3 ubiquitin-protein ligase RNF181 can be used in the present invention in *in vitro* diagnosis of all the diseases specified above for which the E3 ubiquitin-protein ligase RNF181 molecule is or can be indicated. E3 ubiquitin-protein ligase RNF181, is known with the names EC = 06.3.2- and RING finger protein 181, which can be used interchangeably in the context of the present invention.

The UPF0361 protein C3orf37 homolog molecule used herein, consists of 232 amino acids, including all known derivatives and synonyms of this molecule. Said derivatives may be molecules formed by the chemical modification of UPF0361 protein C3orf37 homolog, or constructs formed by the metabolism of this molecule. Amino acid sequence of UPF0361 protein C3orf37 homolog of worms is: wherein, its weight is 26,421 Da.

UPF0361 protein C3orf37 is homologous to the class of RNA-binding proteins and has been found to be produced in embryonic stem cells (Kwon et al., 2013). This protein binds to DNA regions containing 5-hydroxymethylcytosine (5hmC) specifically in stem cells. At this point, the function of the UPF0361 protein C3Fr37 is thought to be a peptidase (Aravind et al., 2013).

In this context, all of the data given in relation to the homologous use of UPF0361 protein C3orf37 in the context of the present invention include derivatives and synonyms of this molecule. In other words, all derivatives and synonyms of the UPF0361 protein C3orf37 homolog can be used in the *in vitro* diagnosis of the diseases for which the UPF0361 protein C3orf37 homologous molecule is / can be indicated in the present invention. UPF0361 protein C3orh37 homolog is also known as HMCES. In the context of the present invention, these names may be used interchangeably.

The term inhibin beta B chain, as used herein, refers to the peptide consisting of 407 amino acids, and all derivatives and synonyms of this molecule known in the art. Said derivatives obtained by chemical modification of the inhibin beta B chain molecules or structures formed by the metabolism of this molecule. Amino acid sequence of B chain is; wherein molecule's weight is 45,122 Da.

Inhibitors inhibit follitropin release from the pituitary gland. Inhibitors play a role in the regulation of various functions depending on hypothalamic and pituitary hormone secretion, gonadal hormone secretion, germ cell development and maturation, erythroid differentiation, insulin secretion, nerve cell survival, embryonic axonal development or bone growth. MRNA synthesis of & quot; CCAAT-enhancer-binding proteins & quot; which are a family of transcription factors involved in cell responses such as cell proliferation, cell growth and differentiation control in hepatocytes, adipocytes, hematopoietic cells, spleen, kidney and many other organs. (Palgi et al., 2000) when administered alone or in combination with BTC to AR42J-B13 cells compared to untreated cells.

In this context, all of the given data relating to the use of inhibin beta B chain in the context of the present invention include derivatives and synonyms of this molecule. In other words, all derivatives and synonyms of the inhibin beta B chain can be used in the *in vitro* diagnosis of any of the diseases mentioned above. Inhibin beta B chain, Inhbb, Inhibin beta-2; activin AB beta polypeptide; inhibin beta B subunit; also known by their names. In the context of the invention, these names can be used interchangeably.

The inventors provided isolation of pancreatic islet-derived mesenchymal stem cells (PI-MSC) at a concentration of 1 mg / ml by administering pancreas of collagenase by perfusion method and shaking at 37 °C for 12 minutes at 100 rpm for digestion.

They were then taken to the culture medium to separate the PI-MSCs from the islets following the isolation of pancreatic islets by applying a ficol gradient at a concentration of 11%, 20%, 23%. Flow cytometric analyzes for characterization of the obtained PI-MSC revealed that 0.18%, 98.1%, 93.2%, 96.5% and CD90% of these cells were CD90, 71% have carried CD106 on their cell surface. They also showed that they exopolate Endoglin, Nestin, Vimentin and Fibronectin as a result of western blot analysis. In addition, the number of these cells was markedly increased in the following days by the WST1 test In another study for characterization, these cells were determined to be specific for β-cells, adipocytes and osteocytes, and to express specific gene expressions by RT-PCR.

The inventors have formed dual culture systems (co-culture) in their own studies. Pancreatic islet-derived mesenchymal stem cells were cultured with primary β cells, and glucotoxicity, lipotoxicity and glucolipotoxicity conditions were established with higher levels of glucose, NEFA (non-esterified fatty acid, palmitic acid) and glucose + NEFA. At the end of the application period (96 hours), some expression levels of some β-cell specific molecules in mesenchymal stem cells from pancreatic islets were measured to determine which cells changed insulin-producing cells. As the final step, proteomic analyzes were performed in secretions (cell media) and the differentiation factors that have the potential to trigger the formation of β-cells have been identified.

The inventors have found that β-cells are killed by apoptosis as a result of co-culture for 96 hours in the presence of 25 mmol / L glucose or 0.4 mmol / L palmitate.

The inventors have found that β-cells are killed by necrosis as a result of co-culturing for 96 hours in the presence of 25 mmol / L glucose or 25 mmol / L glucose + 0.4 mmol / L palmitate.

The inventors performed full proteomic analysis on media by mass spectrophotometry followed by two-dimensional gel electrophoresis in the assay media obtained by co-culture of 96-hour co-culture in the presence of 25 mmol / L glucose or 25 mmol / L glucose + 0.4 mmol / L palmitate. The molecules resulting from this analysis are: E3 ubiquitin-protein ligase RNF181, UPF0361 protein C3orf37 homolog, inhibin beta B chain, aryl-hydrocarbon-interacting-protein-like 1, Ras-related protein Ral-B, STAM- receptor ERR1, Brain-specific angiogenesis inhibitor 1-associated protein 2-like protein 1, NADH dehydrogenase (ubiquinone) iron-sulfur protein 6 mitochondrial, bifunctional protein NCOAT, aldolase B, D-beta-hydroxybutyrate dehydrogenase mitochondrial, ATP synthase subunit b mitochondrial , Betaine-homocysteine S-methyltransferase 1, Guanine deaminase and Alfa-1 acid glycoprotein.

It is therefore contemplated that above-mentioned peptides alone or in combination, or in combination with another agent can be used to obtain beta cells from mesenchymal stem cells for use in the cellular treatment of diabetes.

The inventors have shown that pancreatic islet-derived mesenchymal stem cells differentiate into P-cells as a result of their 96-hour co-culture in the presence of 25 mmol / L glucose or 25 mmol / L glucose + 0.4 mmol / L palmitate. To illustrate this, inventors have obtained β1-cell specific molecules ins1-2, glut2, nkx6.1 and pax6 gene expression products by RT-PCR method and visualized product bands by agarose gel electrophoresis.

The inventors have found that co-cultures in the presence of 25 mmol / L glucose or 25 mmol / L glucose + 0.4 mmol / L palmitate have increased insulin secretion in response to the increased glucose levels of β-cells differentiated from pancreatic islet mesenchymal stem cells they have determined that these cells are functional.

### Examples

### Example 1

### Demonstration of the Morphology of Pancreatic Islets and Mesenchymal Stem Cells Separated Therefrom

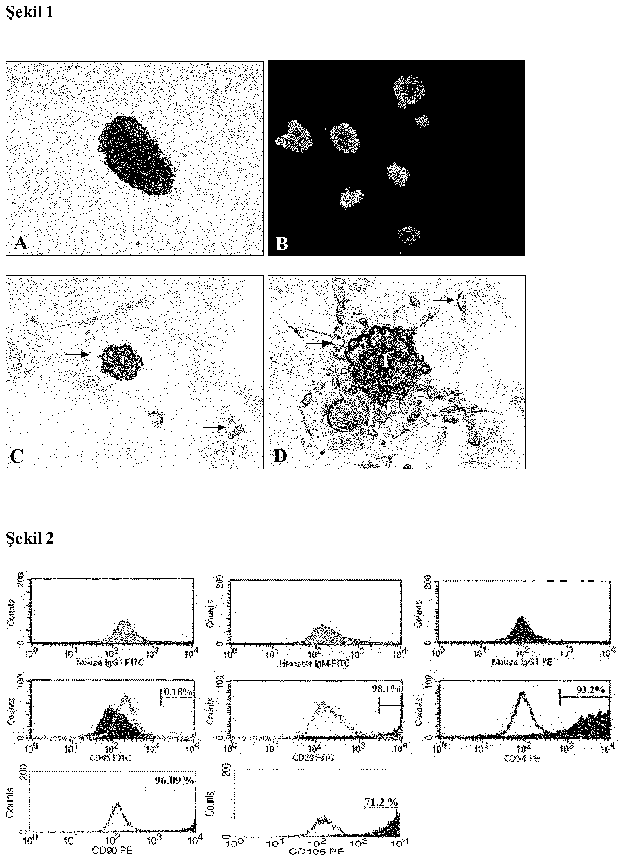

After isolation from adult rats, Langerhans islets were dyed with an insulin marker, dithizone. Moreover, viability of islets was determined by marking with fluorescein diacetate (green) and propidium iodide (red). Almost all cells were detected viable. After 1 and 3 days in culture medium, growing body of fibroblast-like cells (arrow) which are separated from islets, were observed (Figure 1). At the end of 3^{th} day, islets were removed from culture and PI-MSC were observed to be complexified by reproducing.

### Example 2

### Demonstration of Specific Cell Surface Markers of the Stem Cells Originating from Pancreatic Islets

3^{rd} passage pancreatic islet originated stem cells specific cell surface markers are visualized by flow cytometry analysis. According to the obtained results, PI-MSCs express negative control marker; CD45 at rate of %0.18, positive control markers; CD29 at rate of %98.1, CD54 at rate of %93.2, CD90 at rate of %96.09 and CD106 at rate of %71.2 (Figure 2).

### Example 3

### Demonstration of Specific Intracellular Markers of the Stem Cells Originating from Pancreatic Islets

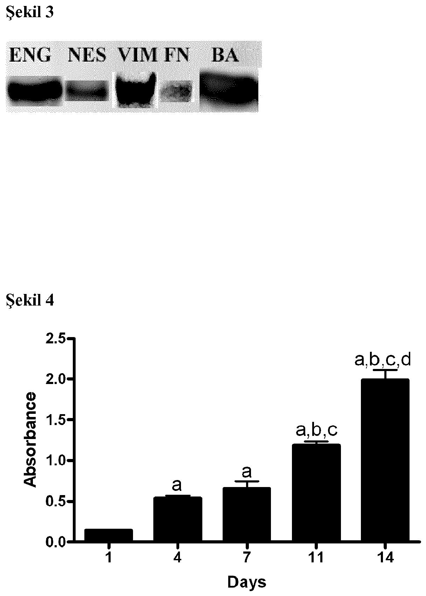

Western blotting showed that 3^{rd} passage pancreatic islet originated stem cells expresses specific intracellular markers such as Endoglin, Nestin, Vimentin, Fibronectin. (Figure 3)

### Example 4

### Demonstration of the Viability of the Stem Cells Originating from Pancreatic Islets

The amount of viable pancreatic islet originated stem cells was demonstrated on 1^{st}, 4^{th}, 7^{th}, 11^{th} and 14^{th} days by WST1 test In the forthcoming days, absorbance values that shows the amount of cell viability, were recorded significant increase (Figure 4). Absorbance values: 0.14±0.01 on 1^{st} day, 0.54±0.03 on 4^{th} day, 0.65±0.09 on 7^{th} day, 01.19±0.04 on 11^{th} day, 1.99±0.12 on 14^{th} day. Results are given as mean ± SEM.

### Example 5

### Demonstration of Differentiation Potential of the Stem Cells Originating from Pancreatic Islets

*In vitro* endocrine, adipogenic and osteogenic differentiation protocols were applied to pancreatic islet originated mesenchymal stem cells and at the end of this process endocrine, adipogenic and osteogenic differentiation parameters were demonstrated by RT-PCR and microscopic technics. Pancreatic β-cell specific molecules: ins-1, ins-2, pdx-1, nkx2-6, pax-6, and glut-2's RT-PCR products were shown in agarose gel for the detection of endocrine differentiation and insulin positive β-cells were immunohistochemically. Adipocyte specific molecules: lpl and ppar2's RT-PCR products were shown in agarose gel and oil vacuoles were histochemically marked with oil red O. Osteocyte specific molecules: sparc, bglap and bgp's RT-PCR products were shown in agarose gel and mineral nodule vacuoles were histochemically marked with Alizarin red S (Figure 5).

### Example 6

### Determination of Apoptosis Rate in Single and Co-Culture Cell Systems Under Glucotoxic, Lipotoxic and Glucolipotoxic Conditions

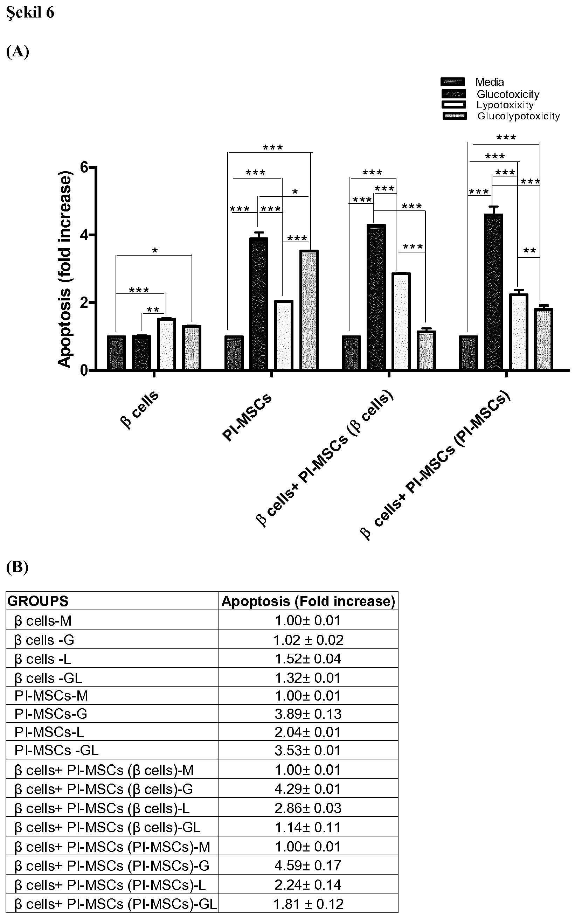

Apoptosis rate of β-cells, pancreatic islet originated stem cells, β-cells taken from β-cells+PI-MSCs coculture system and PI-MSCs taken from β-cells+PI-MSCs coculture system were determined when glucotoxicity, lipotoxicity and glucolipotoxicity conditions applied and even if statistically meaningful changes were determined, they are in low rate. It was determined that apoptosis change of PI-MSC is independent from β-cells on the other hand it was also determined that cells go through apoptosis in higher rates in PI-MSC presence (Figure 6). Based upon these findings, it can be deduced that β-cells die in co-culture condition by partial apoptosis in glucotoxicity condition.

### Example 7

### Determination of Necrosis Rate in Single and Co-Culture Cell Systems Under Glucotoxic, Lipotoxic and Glucolipotoxic Conditions

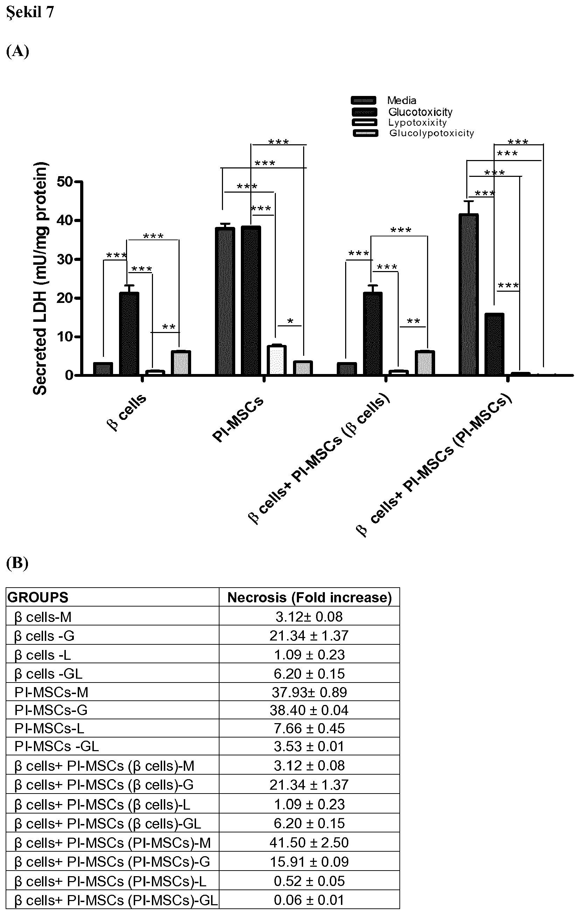

When glucotoxicity, lipotoxicity and glucotoxicity conditions applied, necrosis rate of β-cells, pancreatic islet originated stem cells, β-cells taken from β-cells +PI-MSCs coculture system and PI-MSCs taken from β-cells+PI-MSCs coculture system was determined according to LDH secretion and statistically meaningful significant changes were detected. It was determined that necrotic death of β-cells is independent from PI-MSC on the other hand it was also determined that PI-MSCs go through necrotic death in lower rates in the presence of β-cells (Figure 7). Based upon these findings, it can be deduced that β-cells die dominantly by necrotic way especially in glucotoxicity condition.

### Example 8

### Determination of Proliferation Rate in Single and Co-Culture β-Cell Systems Under Glucotoxic, Lipotoxic and Glucolipotoxic Conditions

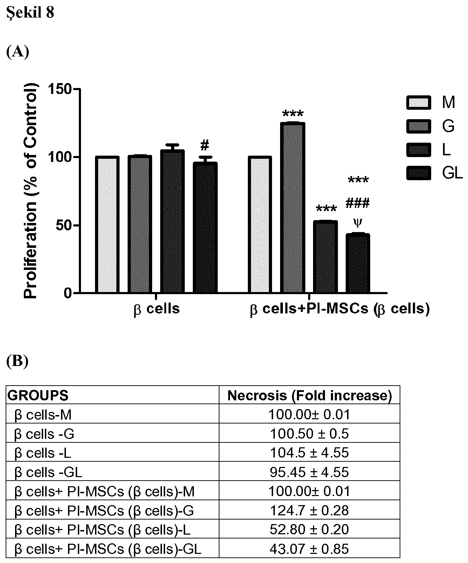

When glucotoxicity, lipotoxicity and glucolipotoxicity conditions applied proliferation rate of β-cells and β-cells that are taken from β-cells + PI-MSCs coculture system are calculated according to BrdU marking. In general, it was detected that β-cell proliferation is not affected from the said conditions, on the other hand while in PI-MSC presence in glucotoxicity condition proliferation is increased significantly, in lipotoxicity and glucolipotoxicity conditions it was detected that it significantly decreases in lipotoxicity and glucolipotoxicity conditions (Figure 8).

### Example 9

### Determination of β-cell Differentiation in PI-MSC Cells of Single and Co-Culture Systems Under Glucotoxicity, Lipotoxic and Glucolipotoxic Conditions

Under glucotoxicity, lipotoxicity and glucolipotoxicity conditions, gene expressions of β-cell specific ins-1, ins-2, pdx-1, glut-2, nkx2-2 and pax-6 in PI-MSCs and PI-MSCs taken from β-cell+ PI-MSC coculture system were detected by RT-PCR technique. Under glucotoxicity and glucolipotoxicity conditions expression of said genes was detected in PI-MSCs taken from β-cells+ PI-MSC coculture system. In glucotoxicity condition, obtained product bands were detected more distinct than glucolipotoxicity condition. Based upon this finding, as well as in glucotoxicity condition with higher, it was observed that in glucolipotoxicity condition differentiation of PI-MSCs into β-cells made a significant contribution to increase in compensatory islet mass.

### Example 10

### Determination of Glucose Responsive Insulin Rate in Single and Co-Culture System Cells Under Glucotoxic, Lipotoxic and Glucolipotoxic Conditions

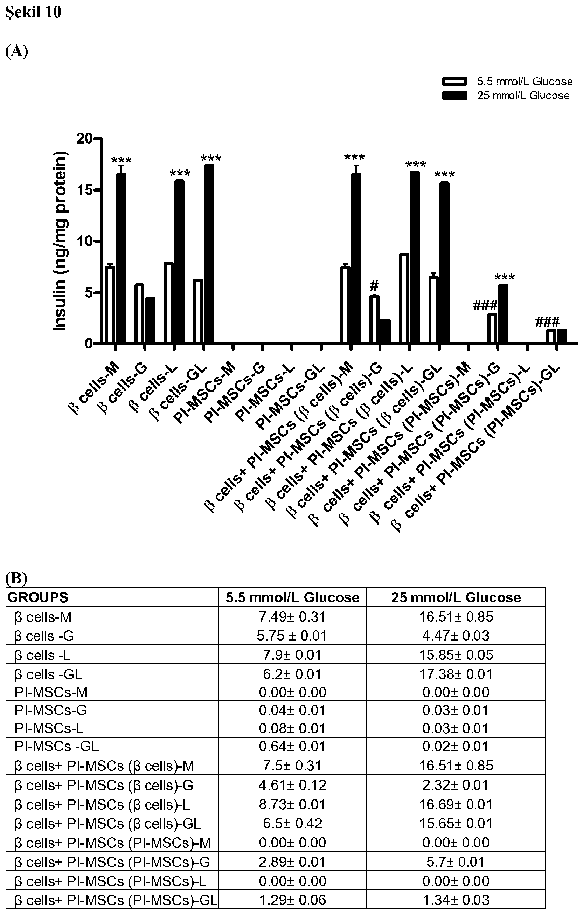

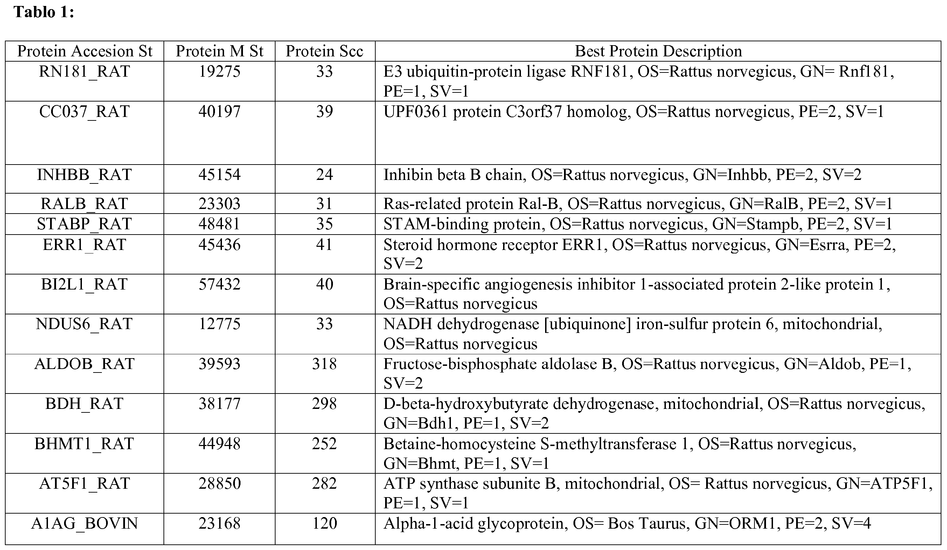

Increased glucose stimulated insulin secretion amounts of β-cells, PI-MSCs, β-cells taken from β-cells+PI-MSCs coculture system, PI-MSCs taken from β-cells+PI-MSCs coculture system were calculated by ELISA method. Similar results were obtained in β-cells in single culture and coculture system. On the other hand, secretion of insulin in no significant amount supports differentiation assays. In PI-MSCs taken from coculture systems insulin levels were also increased compared to increases glucose in glucotoxicity condition (Figure 10). This finding in said glucotoxicity condition shows PI-MSCs differentiate into functional β-cells.

### Example 11

### Media taken from glucotoxicity and glucolipotoxicity treated co-culture system in which β-cell differentiation detected, were analysed by 2D electrophoresis and peptides were determined by mass spectrometry in samples obtained from spots where a difference observed compared to control media

In co-culture system medium where glucotoxicity and glucolipotoxicity were applied, the presence of E3 ubiquitin-protein ligase RNF181, UPF0361 protein C3orf37 homolog, inhibin beta B chain, aryl-hydrocarbon-interacting-protein-like 1, Ras-related protein Ral-B, STAM-binding protein, steroid hormone receptor ERR1, Brain-specific angiogenesis inhibitor 1-associated protein 2-like protein 1, NADH dehydrogenase (ubiquinone) iron-sulfur protein 6 mitochondrial, platelet glycoprotein Ib beta chain, bifunctional protein NCOAT, aldolase B, D-beta-hydroxybutirate dehydrogenase mitochondrial, ATP synthase subunit b mitochondrial, Betaine-homocysteine S-methyltransferase 1 peptids was demonstrated unlikely to control medium (Table 1).

## Claims

1. A method for *in vitro* diagnosis of diabetes, obesity or metabolic diseases associated thereto, wherein the method comprises use of at least one protein molecule as a biomarker selected from the group of:
• E3 ubiquitin-protein ligase RNF181,
• UPF0361 protein C3orf37 homolog,
• Inhibin beta B chain,
or a DNA or mRNA thereof, or a RNA molecule which is selected from miRNAs and IncRNAs that target mRNAs thereof.

2. A method according to claim 1, wherein said disease is Type I or Type II diabetes.

3. A method according to claim 1, wherein the said method comprises detection of the presence of the said biomarkers in a patient's blood, plasma, serum, milk, bronchoalveolar fluid or cerebrospinal fluid.

4. A method according to claim 1, wherein the said method comprises diagnosis by way of ELISA, liquid chromatography, mass spectrometry, western blotting, southern blotting, northern blotting, PCR, RT-PCR, qRT-PCR or microarray assay.

## Patentansprüche

1. Verfahren zur *in-vitro*-Diagnose von Diabetes, Fettleibigkeit oder damit verbundenen Stoffwechselerkrankungen, wobei das Verfahren die Verwendung von mindestens einem Proteinmolekül als Biomarker umfasst, das aus der folgenden Gruppe ausgewählt ist:
• E3-Ubiquitin-Proteinligase RNF181,
• UPF0361 Protein C3orf37 Homolog,
• Inhibin Beta-B-Kette
oder eine DNA oder mRNA hiervon oder ein RNA-Molekül, das aus miRNAs und IncRNAs ausgewählt ist, die auf mRNAs hiervon abzielen.

2. Verfahren nach Anspruch 1, wobei die Krankheit Diabetes vom Typ I oder Typ II ist.

3. Verfahren nach Anspruch 1 wobei das Verfahren den Nachweis des Vorhandenseins der Biomarker in Blut, Plasma, Serum, Milch, bronchoalveolärer Flüssigkeit oder zerebrospinaler Flüssigkeit eines Patienten umfasst.

4. Verfahren nach Anspruch 1, wobei das Verfahren die Diagnose mittels ELISA, Flüssigkeitschromatographie, Massenspektrometrie, Western-Blotting, Southern-Blotting, Northern-Blotting, PCR, RT-PCR, qRT-PCR oder Microarray-Assay umfasst.

## Revendications

1. Procédé de diagnostic *in vitro* d'un diabète, d'une obésité ou de maladies du métabolisme qui y sont associées, lequel procédé comporte l'utilisation, en tant que biomarqueur, d'au moins une molécule protéique choisie dans l'ensemble formé par
- l'ubiquitine-E3-ligase RNF181,
- un homologue de la protéine UPF0361 (C3orf37),
- une chaîne bêta d'inhibine B,
ou d'un ADN ou ARNm correspondant, ou d'une molécule d'ARN qui est choisie parmi les miARN et les lARNnc qui ont pour cibles les ARNm correspondants.

2. Procédé conforme à la revendication 1, dans lequel ladite maladie est un diabète de type I ou de type II.

3. Procédé conforme à la revendication 1, lequel procédé comporte le fait de détecter la présence desdits biomarqueurs dans le sang, le plasma, le sérum, le lait, le liquide broncho-alvéolaire ou le liquide cérébro-spinal d'un patient ou d'une patiente.

4. Procédé conforme à la revendication 1, lequel procédé comporte le fait de réaliser un diagnostic par test ELISA, par chromatographie en phase liquide, par spectrométrie de masse, par test Western blot, Southern blot ou Northern blot, par test de PCR, PCR en temps réel ou PCR quantitative en temps réel, ou par dosage au moyen d'un réseau de micro-puces.
